# EUROPEAN PATENT SPECIFICATION

(11) **EP 3 088 890 B1**
(45) Date of publication and mention of the grant of the patent: **23.09.2020**
(21) Application number: 14874331.3
(22) Date of filing: 25.06.2014
(51) Int. Cl.: G01N 33/53

(54) **METHOD FOR MEASURING SOLUBLE GPC3 PROTEIN**
VERFAHREN ZUR MESSUNG VON LÖSLICHEM GPC3-PROTEIN
MÉTHODE DE MESURE DE PROTÉINE GPC3 SOLUBLE

(30) Priority: 24.12.2013 WO PCT/JP2013/007529
(43) Date of publication of application: 02.11.2016
(73) Proprietor: Chugai Seiyaku Kabushiki Kaisha, Kita-ku Tokyo 115-8543 (JP); JSR Corporation, Minato-ku Tokyo 105-8640 (JP); JSR Life Sciences Corporation, Tokyo 105-8640 (JP)
(72) Inventor: OHTOMO, Toshihiko, Tokyo 103-8324 (JP); AMANO, Jun, Gotemba-shi Shizuoka 412-8513 (JP); ADACHI, Hideki, Gotemba-shi Shizuoka 412-8513 (JP); SUZUKI, Tsukasa, Gotemba-shi Shizuoka 412-8513 (JP); MIZUUCHI, Motoaki, Tokyo 105-8640 (JP); YAMAGUCHI, Tetsuji, Tokyo 105-8640 (JP); WAKUI, Seiki, Tokyo 105-8640 (JP)
(74) Representative: J A Kemp LLP
(86) International application number: PCT/JP2014/003409
(87) International publication number: WO 2015/097928

(56) References cited:
- EP-A1- 2 647 706
- EP-A1- 2 863 224
- WO-A1-2004/038420
- WO-A1-2006/038588
- WO-A1-2013/070468
- WO-A1-2013/181543
- JP-A- 2009 232 848
- US-A1- 2009 060 907
- US-A1- 2010 248 359
- HIPPO YOSHITAKA ET AL: "Identification of soluble NH2-terminal fragment of glypican-3 as a serological marker for early-stage hepatocellular carcinoma", CANCER RESEARCH, AMERICAN ASSOCIATION FOR CANCER RESEARCH, US, vol. 64, no. 7, 1 April 2004 (2004-04-01), pages 2418-2423, XP002424694, ISSN: 0008-5472, DOI: 10.1158/0008-5472.CAN-03-2191
- CHEN MIN ET AL: "Reevaluation of glypican-3 as a serological marker for hepatocellular carcinoma", CLINICA CHIMICA ACTA, ELSEVIER BV, AMSTERDAM, NL, vol. 423, 2 May 2013 (2013-05-02), pages 105-111, XP028558371, ISSN: 0009-8981, DOI: 10.1016/J.CCA.2013.04.026
- HARUYAMA YUKIHIRO ET AL: "High preoperative levels of serum glypican-3 containing N-terminal subunit are associated with poor prognosis in patients with hepatocellular carcinoma after partial hepatectomy", INTERNATIONAL JOURNAL OF CANCER, JOHN WILEY & SONS, INC, US, vol. 137, no. 7, 1 October 2015 (2015-10-01), pages 1643-1651, XP009508562, ISSN: 0020-7136, DOI: 10.1002/IJC.29518

### SEQUENCE LISTING

<110> Chugai Seiyaku Kabushiki Kaisha
   JSR Corporation
   JSR Life Sciences Corporation
<120> Method for determining soluble GPC3 protein
<130> PCG-9039WO
<150> PCT/JP2013/007529
   <151> 2013-12-24
<160> 70
<170> PatentIn version 3.5
<210> 1
   <211> 30
   <212> PRT
   <213> Mus musculus
<400> 1
   Met Ala Gly Thr Val Arg Thr Ala Cys Leu Val Val Ala Met Leu Leu
   1               5                   10                  15
   Ser Leu Asp Phe Pro Gly Gln Ala Gln Pro Pro Pro Pro Pro
               20                  25                  30
<210> 2
   <211> 30
   <212> PRT
   <213> Mus musculus
<400> 2
   Pro Gly Gln Ala Gln Pro Pro Pro Pro Pro Pro Asp Ala Thr Cys His
   1               5                   10                  15
   Gln Val Arg Ser Phe Phe Gln Arg Leu Gln Pro Gly Leu Lys
               20                  25                  30
<210> 3
   <211> 30
   <212> PRT
   <213> Mus musculus
<400> 3
   Phe Phe Gln Arg Leu Gln Pro Gly Leu Lys Trp Val Pro Glu Thr Pro
   1               5                   10                  15
   Val Pro Gly Ser Asp Leu Gln Val Cys Leu Pro Lys Gly Pro
               20                  25                  30
<210> 4
   <211> 30
   <212> PRT
   <213> Mus musculus
<400> 4
   Asp Leu Gln Val Cys Leu Pro Lys Gly Pro Thr Cys Cys Ser Arg Lys
   1               5                   10                  15
   Met Glu Glu Lys Tyr Gln Leu Thr Ala Arg Leu Asn Met Glu
               20                  25                  30
<210> 5
   <211> 30
   <212> PRT
   <213> Mus musculus
<400> 5
   Tyr Gln Leu Thr Ala Arg Leu Asn Met Glu Gln Leu Leu Gln Ser Ala
   1               5                   10                  15
   Ser Met Glu Leu Lys Phe Leu Ile Ile Gln Asn Ala Ala Val
               20                  25                  30
<210> 6
   <211> 30
   <212> PRT
   <213> Mus musculus
<400> 6
   Lys Phe Leu Ile Ile Gln Asn Ala Ala Val Phe Gln Glu Ala Phe Glu
   1               5                   10                  15
   Ile Val Val Arg His Ala Lys Asn Tyr Thr Asn Ala Met Phe
               20                  25                  30
<210> 7
   <211> 30
   <212> PRT
   <213> Mus musculus
<400> 7
   His Ala Lys Asn Tyr Thr Asn Ala Met Phe Lys Asn Asn Tyr Pro Ser
   1               5                   10                  15
   Leu Thr Pro Gln Ala Phe Glu Phe Val Gly Glu Phe Phe Thr
               20                  25                  30
<210> 8
   <211> 30
   <212> PRT
   <213> Mus musculus
<400> 8
   Ala Phe Glu Phe Val Gly Glu Phe Phe Thr Asp Val Ser Leu Tyr Ile
   1               5                   10                  15
   Leu Gly Ser Asp Ile Asn Val Asp Asp Met Val Asn Glu Leu
               20                  25                  30
<210> 9
   <211> 30
   <212> PRT
   <213> Mus musculus
<400> 9
   Ile Asn Val Asp Asp Met Val Asn Glu Leu Phe Asp Ser Leu Phe Pro
   1               5                   10                  15
   Val Ile Tyr Thr Gln Leu Met Asn Pro Gly Leu Pro Asp Ser
               20                  25                  30
<210> 10
   <211> 30
   <212> PRT
   <213> Mus musculus
<400> 10
   Gln Leu Met Asn Pro Gly Leu Pro Asp Ser Ala Leu Asp Ile Asn Glu
   1               5                   10                  15
   Cys Leu Arg Gly Ala Arg Arg Asp Leu Lys Val Phe Gly Asn
               20                  25                  30
<210> 11
   <211> 30
   <212> PRT
   <213> Mus musculus
<400> 11
   Ala Arg Arg Asp Leu Lys Val Phe Gly Asn Phe Pro Lys Leu Ile Met
   1               5                   10                  15
   Thr Gln Val Ser Lys Ser Leu Gln Val Thr Arg Ile Phe Leu
               20                  25                  30
<210> 12
   <211> 30
   <212> PRT
   <213> Mus musculus
<400> 12
   Lys Ser Leu Gln Val Thr Arg Ile Phe Leu Gln Ala Leu Asn Leu Gly
   1               5                   10                  15
   Ile Glu Val Ile Asn Thr Thr Asp His Leu Lys Phe Ser Lys
               20                  25                  30
<210> 13
   <211> 30
   <212> PRT
   <213> Mus musculus
<400> 13
   Asn Thr Thr Asp His Leu Lys Phe Ser Lys Asp Cys Gly Arg Met Leu
   1               5                   10                  15
   Thr Arg Met Trp Tyr Cys Ser Tyr Cys Gln Gly Leu Met Met
               20                  25                  30
<210> 14
   <211> 30
   <212> PRT
   <213> Mus musculus
<400> 14
   Tyr Cys Ser Tyr Cys Gln Gly Leu Met Met Val Lys Pro Cys Gly Gly
   1               5                   10                  15
   Tyr Cys Asn Val Val Met Gln Gly Cys Met Ala Gly Val Val
               20                  25                  30
<210> 15
   <211> 30
   <212> PRT
   <213> Mus musculus
<400> 15
   Val Met Gln Gly Cys Met Ala Gly Val Val Glu Ile Asp Lys Tyr Trp
   1               5                   10                  15
   Arg Glu Tyr Ile Leu Ser Leu Glu Glu Leu Val Asn Gly Met
               20                  25                  30
<210> 16
   <211> 30
   <212> PRT
   <213> Mus musculus
<400> 16
   Leu Ser Leu Glu Glu Leu Val Asn Gly Met Tyr Arg Ile Tyr Asp Met
   1               5                   10                  15
   Glu Asn Val Leu Leu Gly Leu Phe Ser Thr Ile His Asp Ser
               20                  25                  30
<210> 17
   <211> 30
   <212> PRT
   <213> Mus musculus
<400> 17
   Leu Gly Leu Phe Ser Thr Ile His Asp Ser Ile Gln Tyr Val Gln Lys
   1               5                   10                  15
   Asn Ala Gly Lys Leu Thr Thr Thr Ile Gly Lys Leu Cys Ala
               20                  25                  30
<210> 18
   <211> 30
   <212> PRT
   <213> Mus musculus
<400> 18
   Leu Thr Thr Thr Ile Gly Lys Leu Cys Ala His Ser Gln Gln Arg Gln
   1               5                   10                  15
   Tyr Arg Ser Ala Tyr Tyr Pro Glu Asp Leu Phe Ile Asp Lys
               20                  25                  30
<210> 19
   <211> 30
   <212> PRT
   <213> Mus musculus
<400> 19
   Tyr Tyr Pro Glu Asp Leu Phe Ile Asp Lys Lys Val Leu Lys Val Ala
   1               5                   10                  15
   His Val Glu His Glu Glu Thr Leu Ser Ser Arg Arg Arg Glu
               20                  25                  30
<210> 20
   <211> 30
   <212> PRT
   <213> Mus musculus
<400> 20
   Glu Glu Thr Leu Ser Ser Arg Arg Arg Glu Leu Ile Gln Lys Leu Lys
   1               5                   10                  15
   Ser Phe Ile Ser Phe Tyr Ser Ala Leu Pro Gly Tyr Ile Cys
               20                  25                  30
<210> 21
   <211> 30
   <212> PRT
   <213> Mus musculus
<400> 21
   Phe Tyr Ser Ala Leu Pro Gly Tyr Ile Cys Ser His Ser Pro Val Ala
   1               5                   10                  15
   Glu Asn Asp Thr Leu Cys Trp Asn Gly Gln Glu Leu Val Glu
               20                  25                  30
<210> 22
   <211> 30
   <212> PRT
   <213> Mus musculus
<400> 22
   Leu Cys Trp Asn Gly Gln Glu Leu Val Glu Arg Tyr Ser Gln Lys Ala
   1 5 10 15
   Ala Arg Asn Gly Met Lys Asn Gln Phe Asn Leu His Glu Leu
   20 25 30
<210> 23
   <211> 30
   <212> PRT
   <213> Mus musculus
<400> 23
   Met Lys Asn Gln Phe Asn Leu His Glu Leu Lys Met Lys Gly Pro Glu
   1 5 10 15
   Pro Val Val Ser Gln Ile Ile Asp Lys Leu Lys His Ile Asn
   20 25 30
<210> 24
   <211> 30
   <212> PRT
   <213> Mus musculus
<400> 24
   Gln Ile Ile Asp Lys Leu Lys His Ile Asn Gln Leu Leu Arg Thr Met
   1 5 10 15
   Ser Met Pro Lys Gly Arg Val Leu Asp Lys Asn Leu Asp Glu
   20 25 30
<210> 25
   <211> 30
   <212> PRT
   <213> Mus musculus
<400> 25
   Gly Arg Val Leu Asp Lys Asn Leu Asp Glu Glu Gly Phe Glu Ser Gly
   1 5 10 15
   Asp Cys Gly Asp Asp Glu Asp Glu Cys Ile Gly Gly Ser Gly
   20 25 30
<210> 26
   <211> 30
   <212> PRT
   <213> Mus musculus
<400> 26
   Asp Glu Asp Glu Cys Ile Gly Gly Ser Gly Asp Gly Met Ile Lys Val
   1               5                   10                  15
   Lys Asn Gln Leu Arg Phe Leu Ala Glu Leu Ala Tyr Asp Leu
               20                  25                  30
<210> 27
   <211> 30
   <212> PRT
   <213> Mus musculus
<400> 27
   Arg Phe Leu Ala Glu Leu Ala Tyr Asp Leu Asp Val Asp Asp Ala Pro
   1               5                   10                  15
   Gly Asn Ser Gln Gln Ala Thr Pro Lys Asp Asn Glu Ile Ser
               20                  25                  30
<210> 28
   <211> 30
   <212> PRT
   <213> Mus musculus
<400> 28
   Gln Ala Thr Pro Lys Asp Asn Glu Ile Ser Thr Phe His Asn Leu Gly
   1               5                   10                  15
   Asn Val His Ser Pro Leu Lys Leu Leu Thr Ser Met Ala Ile
               20                  25                  30
<210> 29
   <211> 30
   <212> PRT
   <213> Mus musculus
<400> 29
   Thr Phe His Asn Leu Gly Asn Val His Ser Pro Leu Lys Leu Leu Thr
   1               5                   10                  15
   Ser Met Ala Ile Ser Val Val Cys Phe Phe Phe Leu Val His
               20                  25                  30
<210> 30
   <211> 420
   <212> DNA
   <213> Mus musculus
<400> 30
   atggaatgga tctggatctt tctcttcatc ctgtcaggaa ctgcaggtgt ccaatcccag 60
   gttcagctgc agcagtctgg agctgagctg gcgaggcctg gggcttcagt gaaactgtcc 120
   tgcagggctt ctggctacac cttcacaagc tatggtataa gctggatgat gcagagaact 180
   ggacagggcc ttgagtggat tggagagatt tatcctagaa gtggtattac ttactacaat 240
   gagaagttca agggcaaggc cacactgact gcagacaaat cctccagcac agcctacatg 300
   cagctcagca gcctgacatc tgaggactct gcagtctatt tctgtgcaag agatgtctct 360
   gatggttacc tttttcctta ctggggccaa gggactctgg tcactgtctc tgcagccaaa 420
<210> 31
   <211> 387
   <212> DNA
   <213> Mus musculus
<400> 31
   atgagtgtgc ccactcaggt cctggggttg ctgctgctgt ggcttacagg tgccagatgt 60
   gacatccaga tgactcagtc tccagcctcc ctatctgcat ctgtgggaga aactgtcacc 120
   atcacatgtc gaacaagtga gaatatttac agttatttag catggtatca gcagaaacag 180
   ggaaaatctc ctcagctcct ggtctataat gcaaaaacct taccagaagg tgtgccatca 240
   aggttcagtg gcagtggatc aggcacacag ttttctctga agatcaacag cctgcagcct 300
   gaagattttg ggagttatta ctgtcaacat cattatggta ctcctccgac gttcggtgga 360
   ggcaccaagc tggaaatcaa acgggct 387
<210> 32
   <211> 417
   <212> DNA
   <213> Mus musculus
<400> 32
   atgaacttcg ggctcagctt gattttcctt gccctcattt taaaaggtgt ccagtgtgag 60
   gtgcagctgg tggagtctgg gggagacgta gtgagacctg gagggtccct gaaactctcc 120
   tgtgcagcct ctggattcac tttcagtagt tatggcatgt cctgggttcg ccagcttcca 180
   gacaagaggc tggagtgggt cgcaagtgtt ggtaatggag gtagttacag gtactatcca 240
   gagaatttga aggggcggtt caccatctcc agagacaata ccaagaacac cctatacctg 300
   caaattagtg gtctgaagtc tgaggacaca gccatttatt actgtgcaag acggggggct 360
   ttcccgtact tcgatgtctg gggcgcaggg accacggtca ccgtctcctc agccaaa 417
<210> 33
   <211> 390
   <212> DNA
   <213> Mus musculus
<400> 33
   atggattttc aagtgcagat tttcagcttc ctgctaatca gtgcctcagt catagtatcc 60
   agaggacaaa ttgttctcac ccagtctcca gcaatcatgt ctgcatctcc aggggagaag 120
   gtcaccctgg cctgcagtgc cagctcaagt gtaacttaca tgcactggta ccagcagaag 180
   tcaggcacct cccccaaaag atggatttat gaaacatcca aactggcttc tggagtccct 240
   cctcgcttca gtggcagtgg gtctgggacc tcttactctc tcacaatcag caccatggag 300
   gctgaagatg ctgccactta ttactgccaa cagtggagta gtaacccgct cacgttcggt 360
   gctgggacca agctggagct gaaacgggct 390
<210> 34
   <211> 408
   <212> DNA
   <213> Mus musculus
<400> 34
   atgagagtgc tgattctttt gtggctgttc acagcctttc ctggtatcct atctgatgtg 60
   cagcttcagg agtcgggacc tggcctggtg aaaccttctc agtctctgtc cctcacctgc 120
   actgtcactg gctactcaat caccagtgat tctgcctgga actggatccg gcagtttcca 180
   ggaaacaaac tggagtggat ggcctacata atgtacagtg gtatcactag ctacaatcca 240
   tctctcaaaa gtcgaatctc tatcactcga gacacagcca agaaccagtt ctttctgcag 300
   ttgaattctg tgactactga ggactcagcc acatattact gttcacgagg ctactggtac 360
   ttcgatgtct ggggcgcagg gactacggtc accgtctcct cagccaaa 408
<210> 35
   <211> 387
   <212> DNA
   <213> Mus musculus
<400> 35
   atggattttc aggtgcagat tttcagcttc ctgctaatca gtgcctcagt cataatgtcc 60
   agaggacaaa ttgttctcac ccagtctcca gcaatcatgt ctgcatctct aggggaggag 120
   atcaccctaa cctgcagtgc cagctcgagt gtgagttaca tgcactggta ccagcagaag 180
   tcaggcactt ctcccaaact cttgatttat agcacatcca tcctggcttc tggagtccct 240
   tctcgcttca gtggcagtgg gtctgggacc ttttattctc tcacaatcag cagtgtggag 300
   gctgaagatg ctgccgatta ttactgcctt cagtggatta cttatcggac gttcggtgga 360
   ggcaccaagc tggaaatcaa acgggct 387
<210> 36
   <211> 414
   <212> DNA
   <213> Mus musculus
<400> 36
   atgtgttgga gctgtatcat cctcttcctg ttagcaacag ctgcacgtgt gcactcccag 60
   gtccagctgc agcagtctgg ggctgagctg gtggggcctg gggcctcagt gaagatttcc 120
   tgcaaggctt ttggctacac cttcacaaac catcatataa actgggtgaa gcagaggcct 180
   ggacagggcc tggactggat tggatatatt aatccttata atgattatac taactacaac 240
   cagaagttca agggcaaggc cacattgact gtagacaaat cctccagcac agcctatatg 300
   gagcttagca gcctgacatc tgaggactct gcagtctatt actgtgcaag atcagacccc 360
   gcctggtttg cttactgggg ccaagggact ctggtcactg tctctgcagc caaa 414
<210> 37
   <211> 387
   <212> DNA
   <213> Mus musculus
<400> 37
   atgagaccct ccattcagtt cctggggctc ttgttgttct ggcttcatgg tgctcagtgt 60
   gacatccaga tgacacagtc tccatcctca ctgtctgcat ctctgggagg caaagtcacc 120
   atcacttgca aggcaagcca agacattaac aagaatatag cttggtacca acacaagcct 180
   ggaaaaggtc ctaggctgct catatggtac acatatacat tacaaccagg catcccatca 240
   aggttcagtg gaagtggatc tgggagagat tattccttca gcatcagcaa cctggagcct 300
   gaagatattg caacttatta ctgtctacag tatgataatc ttccattcac gttcggcacg 360
   gggacaaaat tggaaataaa acgggct 387
<210> 38
   <211> 121
   <212> PRT
   <213> Mus musculus
<400> 38
   Gln Val Gln Leu Gln Gln Ser Gly Ala Glu Leu Ala Arg Pro Gly Ala
   1 5 10 15
   Ser Val Lys Leu Ser Cys Arg Ala Ser Gly Tyr Thr Phe Thr Ser Tyr
   20 25 30
   Gly Ile Ser Trp Met Met Gln Arg Thr Gly Gln Gly Leu Glu Trp Ile
   35 40 45
   Gly Glu Ile Tyr Pro Arg Ser Gly Ile Thr Tyr Tyr Asn Glu Lys Phe
   50 55 60
   Lys Gly Lys Ala Thr Leu Thr Ala Asp Lys Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Asp Val Ser Asp Gly Tyr Leu Phe Pro Tyr Trp Gly Gln Gly
   100 105 110
   Thr Leu Val Thr Val Ser Ala Ala Lys
   115 120
<210> 39
   <211> 109
   <212> PRT
   <213> Mus musculus
<400> 39
   Asp Ile Gln Met Thr Gln Ser Pro Ala Ser Leu Ser Ala Ser Val Gly
   1               5                   10                  15
   Glu Thr Val Thr Ile Thr Cys Arg Thr Ser Glu Asn Ile Tyr Ser Tyr
               20                  25                  30
   Leu Ala Trp Tyr Gln Gln Lys Gln Gly Lys Ser Pro Gln Leu Leu Val
           35                  40                  45
   Tyr Asn Ala Lys Thr Leu Pro Glu Gly Val Pro Ser Arg Phe Ser Gly
       50                  55                  60
   Ser Gly Ser Gly Thr Gln Phe Ser Leu Lys Ile Asn Ser Leu Gln Pro
   65                  70                  75                  80
   Glu Asp Phe Gly Ser Tyr Tyr Cys Gln His His Tyr Gly Thr Pro Pro
                   85                  90                  95
   Thr Phe Gly Gly Gly Thr Lys Leu Glu Ile Lys Arg Ala
               100                 105
<210> 40
   <211> 120
   <212> PRT
   <213> Mus musculus
<400> 40
   Glu Val Gln Leu Val Glu Ser Gly Gly Asp Val Val Arg Pro Gly Gly
   1 5 10 15
   Ser Leu Lys Leu Ser Cys Ala Ala Ser Gly Phe Thr Phe Ser Ser Tyr
   20 25 30
   Gly Met Ser Trp Val Arg Gln Leu Pro Asp Lys Arg Leu Glu Trp Val
   35 40 45
   Ala Ser Val Gly Asn Gly Gly Ser Tyr Arg Tyr Tyr Pro Glu Asn Leu
   50 55 60
   Lys Gly Arg Phe Thr Ile Ser Arg Asp Asn Thr Lys Asn Thr Leu Tyr
   65 70 75 80
   Leu Gln Ile Ser Gly Leu Lys Ser Glu Asp Thr Ala Ile Tyr Tyr Cys
   85 90 95
   Ala Arg Arg Gly Ala Phe Pro Tyr Phe Asp Val Trp Gly Ala Gly Thr
   100 105 110
   Thr Val Thr Val Ser Ser Ala Lys
   115 120
<210> 41
   <211> 108
   <212> PRT
   <213> Mus musculus
<400> 41
   Gln Ile Val Leu Thr Gln Ser Pro Ala Ile Met Ser Ala Ser Pro Gly
   1 5 10 15
   Glu Lys Val Thr Leu Ala Cys Ser Ala Ser Ser Ser Val Thr Tyr Met
   20 25 30
   His Trp Tyr Gln Gln Lys Ser Gly Thr Ser Pro Lys Arg Trp Ile Tyr
   35 40 45
   Glu Thr Ser Lys Leu Ala Ser Gly Val Pro Pro Arg Phe Ser Gly Ser
   50 55 60
   Gly Ser Gly Thr Ser Tyr Ser Leu Thr Ile Ser Thr Met Glu Ala Glu
   65 70 75 80
   Asp Ala Ala Thr Tyr Tyr Cys Gln Gln Trp Ser Ser Asn Pro Leu Thr
   85 90 95
   Phe Gly Ala Gly Thr Lys Leu Glu Leu Lys Arg Ala
   100 105
<210> 42
   <211> 118
   <212> PRT
   <213> Mus musculus
<400> 42
   Asp Val Gln Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Asp
   20 25 30
   Ser Ala Trp Asn Trp Ile Arg Gln Phe Pro Gly Asn Lys Leu Glu Trp
   35 40 45
   Met Ala Tyr Ile Met Tyr Ser Gly Ile Thr Ser Tyr Asn Pro Ser Leu
   50 55 60
   Lys Ser Arg Ile Ser Ile Thr Arg Asp Thr Ala Lys Asn Gln Phe Phe
   65 70 75 80
   Leu Gln Leu Asn Ser Val Thr Thr Glu Asp Ser Ala Thr Tyr Tyr Cys
   85 90 95
   Ser Arg Gly Tyr Trp Tyr Phe Asp Val Trp Gly Ala Gly Thr Thr Val
   100 105 110
   Thr Val Ser Ser Ala Lys
   115
<210> 43
   <211> 107
   <212> PRT
   <213> Mus musculus
<400> 43
   Gln Ile Val Leu Thr Gln Ser Pro Ala Ile Met Ser Ala Ser Leu Gly
   1               5                   10                  15
   Glu Glu Ile Thr Leu Thr Cys Ser Ala Ser Ser Ser Val Ser Tyr Met
               20                  25                  30
   His Trp Tyr Gln Gln Lys Ser Gly Thr Ser Pro Lys Leu Leu Ile Tyr
           35                  40                  45
   Ser Thr Ser Ile Leu Ala Ser Gly Val Pro Ser Arg Phe Ser Gly Ser
       50                  55                  60
   Gly Ser Gly Thr Phe Tyr Ser Leu Thr Ile Ser Ser Val Glu Ala Glu
   65                  70                  75                  80
   Asp Ala Ala Asp Tyr Tyr Cys Leu Gln Trp Ile Thr Tyr Arg Thr Phe
                   85                  90                  95
   Gly Gly Gly Thr Lys Leu Glu Ile Lys Arg Ala
               100                 105
<210> 44
   <211> 119
   <212> PRT
   <213> Mus musculus
<400> 44
   Gln Val Gln Leu Gln Gln Ser Gly Ala Glu Leu Val Gly Pro Gly Ala
   1               5                   10                  15
   Ser Val Lys Ile Ser Cys Lys Ala Phe Gly Tyr Thr Phe Thr Asn His
               20                  25                  30
   His Ile Asn Trp Val Lys Gln Arg Pro Gly Gln Gly Leu Asp Trp Ile
           35                  40                  45
   Gly Tyr Ile Asn Pro Tyr Asn Asp Tyr Thr Asn Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Arg Ser Asp Pro Ala Trp Phe Ala Tyr Trp Gly Gln Gly Thr Leu
               100                 105                 110
   Val Thr Val Ser Ala Ala Lys
           115
<210> 45
   <211> 109
   <212> PRT
   <213> Mus musculus
<400> 45
   Asp Ile Gln Met Thr Gln Ser Pro Ser Ser Leu Ser Ala Ser Leu Gly
   1               5                   10                  15
   Gly Lys Val Thr Ile Thr Cys Lys Ala Ser Gln Asp Ile Asn Lys Asn
               20                  25                  30
   Ile Ala Trp Tyr Gln His Lys Pro Gly Lys Gly Pro Arg Leu Leu Ile
           35                  40                  45
   Trp Tyr Thr Tyr Thr Leu Gln Pro Gly Ile Pro Ser Arg Phe Ser Gly
       50                  55                  60
   Ser Gly Ser Gly Arg Asp Tyr Ser Phe Ser Ile Ser Asn Leu Glu Pro
   65                  70                  75                  80
   Glu Asp Ile Ala Thr Tyr Tyr Cys Leu Gln Tyr Asp Asn Leu Pro Phe
                   85                  90                  95
   Thr Phe Gly Thr Gly Thr Lys Leu Glu Ile Lys Arg Ala
               100                 105
<210> 46
   <211> 5
   <212> PRT
   <213> Mus musculus
<400> 46
   Ser Tyr Gly Ile Ser
   1               5
<210> 47
   <211> 17
   <212> PRT
   <213> Mus musculus
<400> 47
   Glu Ile Tyr Pro Arg Ser Gly Ile Thr Tyr Tyr Asn Glu Lys Phe Lys
   1               5                   10                  15
   Gly
<210> 48
   <211> 10
   <212> PRT
   <213> Mus musculus
<400> 48
   Asp Val Ser Asp Gly Tyr Leu Phe Pro Tyr
   1               5                   10
<210> 49
   <211> 11
   <212> PRT
   <213> Mus musculus
<400> 49
   Arg Thr Ser Glu Asn Ile Tyr Ser Tyr Leu Ala
   1 5 10
<210> 50
   <211> 7
   <212> PRT
   <213> Mus musculus
<400> 50
   Asn Ala Lys Thr Leu Pro Glu
   1 5
<210> 51
   <211> 9
   <212> PRT
   <213> Mus musculus
<400> 51
   Gln His His Tyr Gly Thr Pro Pro Thr
   1 5
<210> 52
   <211> 5
   <212> PRT
   <213> Mus musculus
<400> 52
   Ser Tyr Gly Met Ser
   1 5
<210> 53
   <211> 17
   <212> PRT
   <213> Mus musculus
<400> 53
   Ser Val Gly Asn Gly Gly Ser Tyr Arg Tyr Tyr Pro Glu Asn Leu Lys
   1 5 10 15
   Gly
<210> 54
   <211> 9
   <212> PRT
   <213> Mus musculus
<400> 54
   Arg Gly Ala Phe Pro Tyr Phe Asp Val
   1 5
<210> 55
   <211> 10
   <212> PRT
   <213> Mus musculus
<400> 55
   Ser Ala Ser Ser Ser Val Thr Tyr Met His
   1 5 10
<210> 56
   <211> 7
   <212> PRT
   <213> Mus musculus
<400> 56
   Glu Thr Ser Lys Leu Ala Ser
   1 5
<210> 57
   <211> 9
   <212> PRT
   <213> Mus musculus
<400> 57
   Gln Gln Trp Ser Ser Asn Pro Leu Thr
   1 5
<210> 58
   <211> 5
   <212> PRT
   <213> Mus musculus
<400> 58
   Asn His His Ile Asn
   1 5
<210> 59
   <211> 17
   <212> PRT
   <213> Mus musculus
<400> 59
   Tyr Ile Asn Pro Tyr Asn Asp Tyr Thr Asn Tyr Asn Gln Lys Phe Lys
   1 5 10 15
   Gly
<210> 60
   <211> 8
   <212> PRT
   <213> Mus musculus
<400> 60
   Ser Asp Pro Ala Trp Phe Ala Tyr
   1 5
<210> 61
   <211> 11
   <212> PRT
   <213> Mus musculus
<400> 61
   Lys Ala Ser Gln Asp Ile Asn Lys Asn Ile Ala
   1 5 10
<210> 62
   <211> 7
   <212> PRT
   <213> Mus musculus
<400> 62
   Tyr Thr Tyr Thr Leu Gln Pro
   1 5
<210> 63
   <211> 19
   <212> PRT
   <213> Mus musculus
<400> 63
   Leu Gln Tyr Asp Asn Leu Pro Phe Thr Phe Gly Thr Gly Thr Lys Leu
   1 5 10 15
   Glu Ile Lys
<210> 64
   <211> 6
   <212> PRT
   <213> Mus musculus
<400> 64
   Ser Asp Ser Ala Trp Asn
   1 5
<210> 65
   <211> 16
   <212> PRT
   <213> Mus musculus
<400> 65
   Tyr Ile Met Tyr Ser Gly Ile Thr Ser Tyr Asn Pro Ser Leu Lys Ser
   1 5 10 15
<210> 66
   <211> 7
   <212> PRT
   <213> Mus musculus
<400> 66
   Gly Tyr Trp Tyr Phe Asp Val
   1 5
<210> 67
   <211> 10
   <212> PRT
   <213> Mus musculus
<400> 67
   Ser Ala Ser Ser Ser Val Ser Tyr Met His
   1 5 10
<210> 68
   <211> 7
   <212> PRT
   <213> Mus musculus
<400> 68
   Ser Thr Ser Ile Leu Ala Ser
   1 5
<210> 69
   <211> 8
   <212> PRT
   <213> Mus musculus
<400> 69
   Leu Gln Trp Ile Thr Tyr Arg Thr
   1 5
<210> 70
   <211> 580
   <212> PRT
   <213> Homo sapiens
<400> 70

## Description

### Technical Field

The present invention relates to a method for assaying soluble GPC3 protein in a test sample, comprising using two different antibodies binding to different epitopes present in the N-terminal region of GPC3 protein.

### Background Art

Deaths caused by hepatocellular carcinoma account for 600,000 deaths each year and are reportedly ranked the 5th most common cancer-related deaths worldwide (Non Patent Literature 1). Most cases of hepatocellular carcinoma die within 1 year after diagnosis as having the disease. Unfortunately, hepatocellular carcinoma patients are frequently diagnosed at a late stage where the patients rarely respond to therapy capable of curing. Medical procedures including chemotherapy, chemoembolization, cauterization, and proton beam therapy are still insufficiently effective for such patients. Many patients exhibit recurrence of the disease, which proceeds rapidly to an advanced stage with vascular invasion and multisite intrahepatic metastasis, resulting in its 5-year survival rate of only 7% (Non Patent Literature 2). Hepatocellular cancer patients with resectable local tumors have relatively good prognosis, but have a 5-year survival rate remaining at 15% to 39% (Non Patent Literature 3).

Glypican-3 (GPC3) is frequently expressed at a high level in liver cancer. It is therefore considered that GPC3 may be useful for the identification of GPC3 functions in liver cancer or as a target for the treatment of liver cancer or a target of the diagnosis of liver cancer.

GPC3 is known to be expressed on cell surface and then processed at the particular site by convertase, phospholipase D, or Notum (Non Patent Literature 4). A method for selecting an HCC patient by assaying GPC3 present in the plasma of patients is disclosed (Patent Literature 1) as a method based on such an event. A diagnostic product for liver cancer comprising an antibody binding to an epitope in a secreted form of GPC3 secreted into plasma, or a method for diagnosing liver cancer using the antibody has been developed (Patent Literatures 2 and 3). Also, a diagnostic product for liver cancer comprising an antibody binding to an epitope in an anchored form of GPC3 still present on cell surface after processing in a tissue specimen or the like isolated from a patient, or a method for diagnosing liver cancer using the antibody has been developed (Patent Literature 4). A method for selecting a prostate cancer patient by measuring a GPC3 level in blood is disclosed, in addition to liver cancer (Patent Literature 5).

All references cited herein are as given below. However, this does not mean that any of these literatures are the prior art relative to the present specification.

### Citation List

### Patent Literature

Patent Literature 1: WO2003/100429
Patent Literature 2: WO2004/038420
Patent Literature 3: WO2004/023145
Patent Literature 4: WO2009/116659
Patent Literature 5: WO2007/081790

### Non Patent Literature

Non Patent Literature 1: Llovet JM, Burroughs A, Bruix J; Lancet (2003), 362, 1907-17
Non Patent Literature 2: Bosch FX, Ribes J, Cleries R; Gastroenterology (2004), 127, S5-16
Non Patent Literature 3: Takenaka K, Kawahara N, Yamamoto K, Kajiyama K, Maeda T, Itasaka H, Shirabe K, Nishizaki T, Yanaga K, Sugimachi K; Arch Surg (1996), 131, 71-6
Non Patent Literature 4: Cheng AL, Chen Z, Tsao CJ, Qin S, Kim JS, et al. Efficacy and safety of sorefanib in patients in the Asia-Pacific region with advanced hepatocellular carcinoma: a phase III randomized, double-blind, placebo-controlled trial. Lancet Oncol. (2009) 10, 25-34

The following further documents are also mentioned:
- US 2009/060907 A1 which refers to an antibody secreted N-terminal peptide of GPC3 present in blood or C-terminal peptide of GPC3;
- Yoshitaka et al (2004) Cancer Research, 64(7): 2418-2423 which refers to identification of a soluble NH₂-terminal fragment of glypican-3 as a serological marker for early-stage hepatocellular carcinoma;
- Min et al (2013) Clinica Chimica Acta, 423: 105-111 which refers to reevaluation of glypican-3 as a serological marker for hepatocellular carcinoma;
- EP 2,863,224 A1 which refers to a kit for diagnosing malignant melanoma;
- WO 2013/181543 A1 which refers to high-affinity monoclonal antibodies to glypican-3 and use thereof;
- US 2010/248359 A1 which refers to an anti-glypican antibody; and
- EP 2,647,706 A1 which refers to an antigen-binding molecule capable of binding to a plurality of antigen molecules repeatedly.

### Summary of Invention

### Technical Problem

No conventional technique is capable of detecting or accurately quantifying a low concentration of soluble GPC3 contained in a healthy subject body fluid. Therefore, early detection at an initial stage in the development of cancer, selection of an anticancer agent used, or post-treatment prognosis cannot be precisely carried out.

An object of the present invention is to provide a highly sensitive assay method for soluble GPC3 capable of quantitatively measuring the concentration of soluble GPC3 contained in a healthy subject body fluid.

### Solution to Problem

The present inventors have conducted diligent studies to attain the object and consequently found that soluble GPC3 protein in a test sample can be assayed highly sensitively by using two different antibodies binding to different epitopes present in the N-terminal region of GPC3 protein.

Specifically, the present invention relates to the following [1] to [7]:
[1] A method for assaying soluble GPC3 protein in a test sample, comprising using two different antibodies binding to different epitopes contained in an amino acid sequence from position 128 to position 357 of GPC3 protein represented by SEQ ID NO: 70,
   wherein the different epitopes are an epitope that is bound by an antibody having a heavy chain variable region comprising a sequence having 80% or higher homology to the sequence represented by SEQ ID NO: 38 and a light chain variable region comprising a sequence having 80% or higher homology to the sequence represented by SEQ ID NO: 39, and an epitope that is bound by an antibody having a heavy chain variable region comprising a sequence having 80% or higher homology to the sequence represented by SEQ ID NO: 40 and a light chain variable region comprising a sequence having 80% or higher homology to the sequence represented by SEQ ID NO: 41, or
   an epitope that is bound by an antibody having a heavy chain variable region comprising a sequence having 80% or higher homology to the sequence represented by SEQ ID NO: 44 and a light chain variable region comprising a sequence having 80% or higher homology to the sequence represented by SEQ ID NO: 45, and an epitope that is bound by an antibody having a heavy chain variable region comprising a sequence having 80% or higher homology to the sequence represented by SEQ ID NO: 42 and a light chain variable region comprising a sequence having 80% or higher homology to the sequence represented by SEQ ID NO: 43.
[2] The method of [1], wherein the two different antibodies are a combination of an antibody having CDR regions identical to CDR regions contained in a heavy chain variable region shown in SEQ ID NO: 38 and CDR regions contained in a light chain variable region shown in SEQ ID NO: 39, and an antibody having CDR regions identical to CDR regions contained in a heavy chain variable region shown in SEQ ID NO: 40 and CDR regions contained in a light chain variable region shown in SEQ ID NO: 41, or
   a combination of an antibody having CDR regions identical to CDR regions contained in a heavy chain variable region shown in SEQ ID NO: 44 and CDR regions contained in a light chain variable region shown in SEQ ID NO: 45, and an antibody having CDR regions identical to CDR regions contained in a heavy chain variable region shown in SEQ ID NO: 42 and CDR regions contained in a light chain variable region shown in SEQ ID NO: 43.
[3] The method of [2], wherein the CDR regions are CDR1, CDR2, and CDR3 regions based on the Kabat numbering.
[4] The method of [1], wherein the two different antibodies are a combination of an antibody having a heavy chain variable region whose CDR1, CDR2, and CDR3 comprise the amino acid sequences represented by SEQ ID NOs: 46, 47, and 48, respectively, and having a light chain variable region whose CDR1, CDR2, and CDR3 comprise the amino acid sequences represented by SEQ ID NOs: 49, 50, and 51, respectively, and an antibody having a heavy chain variable region whose CDR1, CDR2, and CDR3 comprise the amino acid sequences represented by SEQ ID NOs: 52, 53, and 54, respectively, and having a light chain variable region whose CDR1, CDR2, and CDR3 comprise the amino acid sequences represented by SEQ ID NOs: 55, 56, and 57, respectively, or
   a combination of an antibody having a heavy chain variable region whose CDR1, CDR2, and CDR3 comprise the amino acid sequences represented by SEQ ID NOs: 58, 59, and 60, respectively, and having a light chain variable region whose CDR1, CDR2, and CDR3 comprise the amino acid sequences represented by SEQ ID NOs: 61, 62, and 63, respectively, and an antibody having a heavy chain variable region whose CDR1, CDR2, and CDR3 comprise the amino acid sequences represented by SEQ ID NOs: 64, 65, and 66, respectively, and having a light chain variable region whose CDR1, CDR2, and CDR3 comprise the amino acid sequences represented by SEQ ID NOs: 67, 68, and 69, respectively.
[5] The method of [1], wherein the two different antibodies are a combination of an antibody having a heavy chain variable region shown in SEQ ID NO: 38 and a light chain variable region shown in SEQ ID NO: 39, and an antibody having a heavy chain variable region shown in SEQ ID NO: 40 and a light chain variable region shown in SEQ ID NO: 41, or
   a combination of an antibody having a heavy chain variable region shown in SEQ ID NO: 44 and a light chain variable region shown in SEQ ID NO: 45, and an antibody having a heavy chain variable region shown in SEQ ID NO: 42 and a light chain variable region shown in SEQ ID NO: 43.
[6] The method of any one of [1] to [5], wherein any one of the two different antibodies is bound with a magnetic particle.
[7] The method according to any one of [1] to [6], wherein the test sample is a tissue sample, a whole blood sample, a plasma sample, or a serum sample isolated from a human.

### Advantageous Effects of Invention

According to the present invention, soluble GPC3 protein in a test sample can be assayed conveniently and highly sensitively by using two different antibodies binding to different epitopes present in the N-terminal region of GPC3 protein. As a result, early detection at an initial stage in the development of cancer, selection of an anticancer agent used, and post-treatment prognosis can be precisely carried out.

### Brief Description of Drawings

[Figure 1] Figure 1 shows results of subjecting mouse antibodies obtained by screening to Western blotting for GPC3 and a C fragment and N fragment thereof under reductive conditions.
[Figure 2] Figure 2 shows results of subjecting GT30, GT114, GT607, and GT165 antibodies to Western blotting under reduction of pellets of CHO cells expressing either GPC3 or each fragment thereof (AW2, AW3, and AW5).
[Figure 3] Figure 3 shows results of a binding test between the GT30, GT114, GT607, or GT165 antibody and each fragment of GPC3.
[Figure 4] Figure 4 schematically shows a GT30, GT114, GT607, or GT165 antibody-binding region on GPC3.
[Figure 5] Figure 5 shows a GT30-binding region in the conformation of human GPC3.
[Figure 6] Figure 6 shows a GT165-binding region in the conformation of human GPC3.
[Figure 7] Figure 7 shows a GT607-binding region in the conformation of human GPC3.
[Figure 8] Figure 8 shows a GT114-binding region in the conformation of human GPC3.
[Figure 9] Figure 9 shows the GT30-, GT165-, GT607- and GT114-binding regions overlaid on each other in the conformation of human GPC3.
[Figure 10] Figure 10 is a graph showing the detection limit and the quantification limit of each assay system.

### Description of Embodiments

### Definition

In the present specification, the chemical terms and the technical terms used in relation to the present invention have meanings that are generally understood by those skilled in the art, unless otherwise specified.

### Indefinite article

In the present invention, the indefinite article "a" or "an" refers to one or two or more (i.e., at least one) grammatical subject(s) of the indefinite article. For example, "a factor" means one factor or two or more factors.

### Amino acid

In the present specification, each amino acid is indicated by a one-letter or three-letter code, or both, as indicated by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

### Alteration of amino acid

A method known in the art such as site-directed mutagenesis (Kunkel et al., Proc. Natl. Acad. Sci. USA (1985) 82, 488-492) or overlap extension PCR can be appropriately adopted for the alteration of an amino acid in the amino acid sequence of an antigen-binding molecule. A plurality of methods known in the art can also be adopted as alteration methods for amino acids to be substituted by amino acids other than natural amino acids (Annu. Rev. Biophys. Biomol. Struct. (2006) 35, 225-249; and Proc. Natl. Acad. Sci. U.S.A. (2003) 100 (11), 6353-6357). For example, a cell-free translation system (Clover Direct (Protein Express Co., Ltd.)) is preferably used which contains tRNA in which a non-natural amino acid is bound with amber suppressor tRNA complementary to a stop codon UAG (amber codon).

In the present specification, the term "and/or" used for indicating an amino acid alteration site includes every combination in which "and" and "or" are appropriately combined. Specifically, the phrase "amino acids at positions 43, 52, and/or 105 are substituted" includes the following variations of amino acid alteration:
(a) position 43, (b) position 52, (c) position 105, (d) positions 43 and 52, (e) positions 43 and 105, (f) positions 52 and 105, and (g) positions 43, 52, and 105.

### Numbering of CDR

The amino acid positions assigned to antibody CDRs can be specified according to a method known in the art and can be specified according to, for example, Kabat (Sequences of Proteins of Immunological Interest, National Institute of Health, Bethesda, Md., 1987 and 1991).

### Test sample

The term "test sample" refers to a sample of a tissue or fluid isolated from a subject. Examples of such a sample include plasma, serum, spinal fluid, lymph, external sections of the skin, the respiratory tract, the intestinal tract, and the genitourinary tract, tear, saliva, sputum, milk, urine, whole blood or any blood fraction, blood derivatives, blood cells, tumors, nervous tissues, organs or any type of tissue, any sample obtained by lavage (e.g., samples derived from the bronchi), and samples of components constituting cell cultures *in vitro.*

The concentration of soluble GPC3 can be measured in a biological sample (test sample) isolated from a patient. For example, the concentration of soluble GPC3 can be measured in a sample of whole blood or a sample of a blood fraction such as serum or plasma (in the present specification, also referred to as a whole blood sample, a serum sample, or a plasma sample, respectively). In a non-limiting embodiment, the concentration of soluble GPC3 in the whole blood sample, the serum sample, or the plasma sample of a patient can be measured using, for example, commercially available Human Glypican-3 ELISA kit (BioMosaic Inc.) or Enzyme-linked Immunosorbent Assay Kit For Glypican 3 (GPC3) (USCN Life Science Inc.) and an EDTA-treated whole blood sample, serum sample, or plasma sample.

The term "isolated" refers to causing "artificial" change from a natural state, i.e., shifting and/or removing a naturally occurring substance from its original environment. The term "isolated" means that, for example, a polynucleotide or a polypeptide present in an organism is unisolated, whereas the same polynucleotide or polypeptide thereas is isolated when separated from a material present with the polynucleotide or the polypeptide in a natural state.

### Soluble GPC3

"soluble GPC3" refers to a soluble form of GPC3 unanchored to GPC3-expressing cells and includes fragments of a secreted form of GPC3 that can be easily dissociated from GPC3 anchored to GPC3-expressing cells under particular conditions *in vivo* or *in vitro.* In a non-limiting embodiment, examples of the "soluble GPC3" can include a polypeptide from the amino terminus to position 358 in GPC3 defined by SEQ ID NO: 70, a polypeptide from the amino terminus to position 374 in GPC3 defined by SEQ ID NO: 70, a GPC3 polypeptide liberated by the degradation of a GPI anchor present at the carboxy terminus, and their fragments (Patent Literature 2). Those skilled in the art can appropriately select an approach known in the art for determining the structure of soluble GPC3. A method therefor that may be appropriately used which involves, for example, directly detecting soluble GPC3 present in the serum or the plasma of a patient or a model animal by the method described in Patent Literature 2 and analyzing its structure, or which involves, for example, allowing an enzyme dissociating soluble GPC3, such as convertase, phospholipase D, or Notum, to act on GPC3 expressed in cells cultured *in vitro,* detecting the resulting soluble GPC3, and analyzing its structure (e.g., J. Cell. Biol. (2003) 163 (3), 625-635).

### Method for measuring soluble GPC3 concentration

In the present invention, the soluble GPC3 concentration is measured by an immunological method using two different antibodies binding to different epitopes contained in an amino acid sequence from position 128 to position 357, preferably position 219 to position 357. In particular, the present invention provides a method for assaying soluble GPC3 protein in a test sample, comprising using two different antibodies binding to different epitopes contained in an amino acid sequence from position 128 to position 357 of GPC3 protein represented by SEQ ID NO: 70,
wherein the different epitopes are an epitope that is bound by an antibody having a heavy chain variable region comprising a sequence having 80% or higher homology to the sequence represented by SEQ ID NO: 38 and a light chain variable region comprising a sequence having 80% or higher homology to the sequence represented by SEQ ID NO: 39, and an epitope that is bound by an antibody having a heavy chain variable region comprising a sequence having 80% or higher homology to the sequence represented by SEQ ID NO: 40 and a light chain variable region comprising a sequence having 80% or higher homology to the sequence represented by SEQ ID NO: 41, or
an epitope that is bound by an antibody having a heavy chain variable region comprising a sequence having 80% or higher homology to the sequence represented by SEQ ID NO: 44 and a light chain variable region comprising a sequence having 80% or higher homology to the sequence represented by SEQ ID NO: 45, and an epitope that is bound by an antibody having a heavy chain variable region comprising a sequence having 80% or higher homology to the sequence represented by SEQ ID NO: 42 and a light chain variable region comprising a sequence having 80% or higher homology to the sequence represented by SEQ ID NO: 43.

Preferred examples of the method for assaying soluble GPC3 include enzyme immunoassay (ELISA or EIA), fluorescence immunoassay (FIA), radioimmunoassay (RIA), luminescence immunoassay (LIA), immunoenzymatic technique, fluorescent antibody technique, immunochromatography, immunoturbidimetry, latex turbidimetry, and latex agglutination assay. In the immunological method of the present invention, the soluble GPC3 can be assayed by procedures of manual operation or using an apparatus such as an analyzer.

The immunological method according to the present invention can be carried out according to a method known in the art, for example, sandwich technique. For example, a first antibody immobilized on a carrier, a biological sample, and a second antibody modified with a labeling material are reacted simultaneously or sequentially. This reaction forms a complex of the first antibody immobilized on a carrier, soluble GPC3, and the second antibody modified with a labeling material. The labeling material conjugated with the second antibody contained in this complex can be quantified to measure the amount (concentration) of the soluble GPC3 contained in the biological sample.

In the case of, for example, the enzyme immunoassay, a first antibody-immobilized microplate, serially diluted biological samples, a second antibody modified with an enzyme such as HRP, a washing buffer, and a solution containing a substrate reactive with the enzyme such as HRP are preferably used. In a non-limiting embodiment, the assay can involve reacting the enzyme modifying the second antibody under the optimum conditions thereof with the substrate, and measuring the amount of the resulting enzymatic reaction product by an optical method or the like. In the case of the fluorescence immunoassay, a first antibody-immobilized optical waveguide, serially diluted biological samples, a second antibody modified with a fluorescent material, and a washing buffer can be preferably used. In a non-limiting embodiment, the assay can involve irradiating the fluorescent material modifying the second antibody with excitation light to emit fluorescence, the intensity of which is then measured.

The radioimmunoassay involves measuring the amount of radiation from a radioactive substance. The luminescence immunoassay involves measuring luminescence intensity derived from a luminescent reaction system. For example, the immunoturbidimetry, the latex turbidimetry, or the latex agglutination assay involves measuring transmitted light or scattering light by an endpoint or rate method. The immunochromatography, for example, which is based on visual observation, involves visually measuring the color of the labeling material appearing on a test line. Alternatively, an instrument such as an analyzer may be appropriately used instead of this visual measurement.

In the immunological method of the present invention, the first antibody to be immobilized on a carrier can be adsorbed or bound to the carrier by a method such as physical adsorption, chemical binding, or a combination thereof. A method known in the art can be appropriately used as the method for immobilizing the antibody by physical adsorption. Examples thereof include a method which involves contacting the antibody with the carrier by mixing in a solution such as a buffer solution, and a method which involves contacting the antibody dissolved in a buffer or the like with the carrier. Alternatively, the antibody may be immobilized onto the carrier by chemical binding. Examples thereof include a method which involves mixing and contacting the antibody and the carrier with a divalent cross-linking reagent such as glutaraldehyde, carbodiimide, imide ester, or maleimide to react the reagent with amino groups, carboxyl groups, thiol groups, aldehyde groups, hydroxy groups, or the like in both of the antibody and the carrier. Such immobilization may require treatment for suppressing nonspecific reaction or the natural aggregation or the like of the antibody-immobilized carrier. In such a case, the aftertreatment of the immobilization can be carried out by a method known in the art. Examples thereof include a method which involves coating the surface or the inner wall of the antibody-immobilized carrier by contact with, for example, a protein (e.g., bovine serum albumin (BSA), casein, gelatin, egg albumin, or a salt thereof), a surfactant, or skimmed milk.

In the immunological method of the present invention, the second antibody to be modified with a labeling material can be adsorbed or bound to the labeling material by a method such as physical adsorption, chemical binding, or a combination thereof. A method known in the art can be appropriately used as the method for binding the labeling material to the antibody by physical adsorption. Examples of thereof include a method which involves contacting the antibody with the labeling material by mixing in a solution such as a buffer solution, and a method which involves contacting the antibody dissolved in a buffer or the like with the labeling material. When the labeling material is, for example, gold colloid or latex, the physical adsorption method is effective. The antibody can be mixed and contacted with the gold colloid in a buffer to obtain a gold colloid-labeled antibody. Alternatively, the antibody may be modified with the labeling material by chemical binding. Examples thereof include a method which involves contacting and mixing the antibody and the labeling material with a divalent cross-linking reagent such as glutaraldehyde, carbodiimide, imide ester, or maleimide to react the reagent with amino groups, carboxyl groups, thiol groups, aldehyde groups, hydroxy groups, or the like in both of the antibody and the labeling material. When the labeling material is, for example, a fluorescent material, an enzyme, or a chemiluminescent material, the chemical binding method is effective. Such modification may require treatment for suppressing nonspecific reaction or the natural aggregation or the like of the antibody modified with the labeling material. In such a case, the aftertreatment of the labeling can be carried out by a method known in the art. Examples thereof include a method which involves coating the labeling material-bound antibody by contacting with, for example, a protein (e.g., bovine serum albumin (BSA), casein, gelatin, egg albumin, or a salt thereof), a surfactant, or skimmed milk.

For example, peroxidase (POD), alkaline phosphatase (ALP), β-galactosidase, urease, catalase, glucose oxidase, lactate dehydrogenase, or amylase can be used as the labeling material for the enzyme immunoassay. For example, fluorescein isothiocyanate, tetramethylrhodamine isothiocyanate, substituted rhodamine isothiocyanate, dichlorotriazine isothiocyanate, cyanine, or merocyanine can be used for the fluorescence immunoassay. For example, tritium, iodine 125, or iodine 131 can be used for the radioimmunoassay. For example, a luminol system, a luciferase system, an acridinium ester system, or a dioxetane compound system can be used for the luminescence immunoassay. Also, fine particles made of a material such as polystyrene, a styrene-styrene sulfonate copolymer, an acrylonitrile-butadiene-styrene copolymer, a vinyl chloride-acrylic acid ester copolymer, a vinyl acetate-acrylic acid copolymer, polyacrolein, a styrene-methacrylic acid copolymer, a styrene-glycidyl (meth)acrylate copolymer, a styrenebutadiene copolymer, a methacrylic acid polymer, an acrylic acid polymer, latex, gelatin, liposome, a microcapsule, silica, alumina, carbon black, a metal compound, a metal, a metal colloid, a ceramic, or a magnetic substance can be used for the immunochromatography, the immunoturbidimetry, the latex turbidimetry, or the latex agglutination assay.

A solid-phase carrier in the form of, for example, beads, a microplate, a test tube, a stick, a membrane, or a test pieces made of a material such as polystyrene, polycarbonate, polyvinyltoluene, polypropylene, polyethylene, polyvinyl chloride, nylon, polymethacrylate, polyacrylamide, latex, liposome, gelatin, agarose, cellulose, Sepharose, glass, a metal, a ceramic, or a magnetic substance can be appropriately used as the carrier in the immunological method of the present invention. Particularly, a magnetic substance such as magnetic particles is preferred. Polymer particles containing a magnetic substance or a superparamagnetic substance are preferred as the magnetic particles. Magnetic particles are more preferred in which a magnetic substance layer containing at least one of Fe₂O₃ and Fe₃O₄ is formed on the surface of a core particle and a polymer layer is further formed on the magnetic substance layer.

### Two different antibodies

The "two different antibodies" used in the present invention bind to "different epitopes" contained in an amino acid region from position 128 to position 357 of GPC3. The "different epitopes" are an epitope that is bound by an antibody having a heavy chain variable region comprising a sequence having 80% or higher homology to the sequence represented by SEQ ID NO: 38 and a light chain variable region comprising a sequence having 80% or higher homology to the sequence represented by SEQ ID NO: 39, and an epitope that is bound by an antibody having a heavy chain variable region comprising a sequence having 80% or higher homology to the sequence represented by SEQ ID NO: 40 and a light chain variable region comprising a sequence having 80% or higher homology to the sequence represented by SEQ ID NO: 41, or
an epitope that is bound by an antibody having a heavy chain variable region comprising a sequence having 80% or higher homology to the sequence represented by SEQ ID NO: 44 and a light chain variable region comprising a sequence having 80% or higher homology to the sequence represented by SEQ ID NO: 45, and an epitope that is bound by an antibody having a heavy chain variable region comprising a sequence having 80% or higher homology to the sequence represented by SEQ ID NO: 42 and a light chain variable region comprising a sequence having 80% or higher homology to the sequence represented by SEQ ID NO: 43.
Specific examples thereof can include a combination of an epitope on GPC3 that is bound by a GT30 antibody described in Examples and an epitope on GPC3 that is bound by a GT607 antibody described therein, and a combination of an epitope on GPC3 that is bound by a GT114 antibody described therein and an epitope on GPC3 that is bound by a GT165 antibody described therein. Examples of such a combination of the different epitopes include a combination of an epitope (for GT30 and GT165) comprising at least one amino acid selected from amino acids at positions 337, 339, 340, and 344 of the GPC3 protein and an epitope (for GT607 and GT114) comprising at least one amino acid selected from amino acids at positions 221, 298, 301, 302, 305, 308, and 309 of the GPC3 protein. Alternative examples thereof include a combination of an epitope (for GT30) comprising at least one amino acid selected from amino acids at positions 343, 346, 347, 348, 349, and 350 of the GPC3 protein and an epitope (for GT607) comprising at least one amino acid selected from amino acids at positions 297, 300, 304, 306, 311, 312, 313, 314, and 315 of the GPC3 protein, and a combination of an epitope (for GT165) comprising at least one amino acid selected from amino acids at positions 128, 129, 131, 132, 133, 134, 135, 171, 208, 209, 210, 211, 212, 214, 215, 218, 322, 325, 326, 328, 329, 330, 332, 333, 335, 336, and 338 of the GPC3 protein and an epitope (for GT114) comprising at least one amino acid selected from amino acids at positions 220, 228, 231, 232, 235, 291, 294, and 295 of the GPC3 protein. Further examples thereof can include a combination of an epitope (for GT30) comprising at least one amino acid selected from amino acids at positions 337, 339, 340, and 344 of the GPC3 protein and comprising at least one amino acid selected from amino acids at positions 341, 343, 346, 347, 348, 349, and 350 thereof, and an epitope (for GT607) comprising at least one amino acid selected from amino acids at positions 221, 298, 301, 302, 305, 308, and 309 of the GPC3 protein and comprising at least one amino acid selected from amino acids at positions 297, 300, 304, 306, 311, 312, 313, 314, and 315 thereof, and a combination of an epitope (for GT165) comprising at least one amino acid selected from amino acids at positions 337, 339, 340, and 344 of the GPC3 protein and comprising at least one amino acid selected from amino acids at positions 128, 129, 131, 132, 133, 134, 135, 171, 208, 209, 210, 211, 212, 214, 215, 218, 322, 325, 326, 328, 329, 330, 332, 333, 335, 336, and 338 thereof, and an epitope (for GT114) comprising at least one amino acid selected from amino acids at positions 221, 298, 301, 302, 305, 308, and 309 of the GPC3 protein and comprising at least one amino acid selected from amino acids at positions 220, 228, 231, 232, 235, 291, 294, and 295 thereof.

The "two different antibodies" are not particularly limited as long as the different antibodies recognize different epitopes in the region described above and are in a relationship that does not mutually inhibit their binding to the soluble GPC3. Specific examples of the "two different antibodies" used in the present invention can include a combination of a GT30 antibody and a GT607 antibody, and a combination of a GT165 antibody and a GT114 antibody.

### GT30 antibody

The GT30 antibody recognizes an epitope comprising at least one amino acid selected from amino acids at positions 337, 339, 340, 341, 344, 343, 346, 347, 348, 349, and 350 of the GPC3 protein. The GT30 antibody has a heavy chain variable region shown in SEQ ID NO: 38 and a light chain variable region shown in SEQ ID NO: 39 and has heavy chain CDR1, heavy chain CDR2, and heavy chain CDR3 comprising the amino acid sequences represented by SEQ ID NO: 46, SEQ ID NO: 47, and SEQ ID NO: 48, respectively, and light chain CDR1, light chain CDR2, and light chain CDR3 comprising the amino acid sequences represented by SEQ ID NO: 49, SEQ ID NO: 50, and SEQ ID NO: 51, respectively (these sequences of CDR1, CDR2, and CDR3 are based on the Kabat numbering).

### GT607 antibody

The GT607 antibody recognizes an epitope comprising at least one amino acid selected from amino acids at positions 221, 298, 301, 302, 305, 308, 309, 297, 300, 304, 306, 311, 312, 313, 314, and 315 of the GPC3 protein. The GT607 antibody has a heavy chain variable region shown in SEQ ID NO: 40 and a light chain variable region shown in SEQ ID NO: 41 and has heavy chain CDR1, heavy chain CDR2, and heavy chain CDR3 comprising the amino acid sequences represented by SEQ ID NO: 52, SEQ ID NO: 53, and SEQ ID NO: 54, respectively, and light chain CDR1, light chain CDR2, and light chain CDR3 comprising the amino acid sequences represented by SEQ ID NO: 55, SEQ ID NO: 56, and SEQ ID NO: 57, respectively (these sequences of CDR1, CDR2, and CDR3 are based on the Kabat numbering).

### GT165 antibody

The GT165 antibody recognizes an epitope comprising at least one amino acid selected from amino acids at positions 337, 339, 340, 344, 128, 129, 131, 132, 133, 134, 135, 171, 208, 209, 210, 211, 212, 214, 215, 218, 322, 325, 326, 328, 329, 330, 332, 333, 335, 336, and 338 of the GPC3 protein. The GT165 antibody has a heavy chain variable region shown in SEQ ID NO: 44 and a light chain variable region shown in SEQ ID NO: 45 and has heavy chain CDR1, heavy chain CDR2, and heavy chain CDR3 comprising the amino acid sequences represented by SEQ ID NO: 58, SEQ ID NO: 59, and SEQ ID NO: 60, respectively, and light chain CDR1, light chain CDR2, and light chain CDR3 comprising the amino acid sequences represented by SEQ ID NO: 61, SEQ ID NO: 62, and SEQ ID NO: 63, respectively (these sequences of CDR1, CDR2, and CDR3 are based on the Kabat numbering).

### GT114 antibody

The GT114 antibody recognizes an epitope comprising at least one amino acid selected from amino acids at positions 221, 298, 301, 302, 305, 308, and 309 of the GPC3 protein and comprising at least one amino acid selected from amino acids at positions 220, 228, 231, 232, 235, 291, 294, and 295 thereof. The GT114 antibody has a heavy chain variable region shown in SEQ ID NO: 42 and a light chain variable region shown in SEQ ID NO: 43 and has heavy chain CDR1, heavy chain CDR2, and heavy chain CDR3 comprising the amino acid sequences represented by SEQ ID NO: 64, SEQ ID NO: 65, and SEQ ID NO: 66, respectively, and light chain CDR1, light chain CDR2, and light chain CDR3 comprising the amino acid sequences represented by SEQ ID NO: 67, SEQ ID NO: 68, and SEQ ID NO: 69, respectively (these sequences of CDR1, CDR2, and CDR3 are based on the Kabat numbering).

Hereinafter, the nucleotide sequences and the amino acid sequences of the heavy chain and light chain variable regions of each antibody will be described:
Nucleotide sequence of GT30 H chain (SEQ ID NO: 30)
Nucleotide sequence of GT30 L chain (SEQ ID NO: 31)
Nucleotide sequence of GT607 H chain (SEQ ID NO: 32)
Nucleotide sequence of GT607 L chain (SEQ ID NO: 33)
Nucleotide sequence of GT114 H chain (SEQ ID NO: 34)
Nucleotide sequence of GT114 L chain (SEQ ID NO: 35)
Nucleotide sequence of GT165 H chain (SEQ ID NO: 36)
Nucleotide sequence of GT165 L chain (SEQ ID NO: 37)
Amino acid sequence of GT30 H chain variable region (SEQ ID NO: 38)
Amino acid sequence of GT30 L chain variable region (SEQ ID NO: 39)
Amino acid sequence of GT607 H chain variable region (SEQ ID NO: 40)
Amino acid sequence of GT607 L chain variable region (SEQ ID NO: 41)
Amino acid sequence of GT114 H chain variable region (SEQ ID NO: 42)
Amino acid sequence of GT114 L chain variable region (SEQ ID NO: 43)
Amino acid sequence of GT165 H chain variable region (SEQ ID NO: 44)
Amino acid sequence of GT165 L chain variable region (SEQ ID NO: 45)

Hereinafter, the CDR sequences of each antibody will be described.
GT30 H chain CDR sequences

| | | |
|---|---|---|
| CDR1 (SEQ ID NO: 46) | CDR2 (SEQ ID NO: 47) | CDR3 (SEQ ID NO: 48) |
| SYGIS | EIYPRSGITYYNEKFKG | DVSDGYLFPY |

Amino acid sequence of GT30 L chain variable region

| | | |
|---|---|---|
| CDR1 (SEQ ID NO: 49) | CDR2 (SEQ ID NO: 50) | CDR3 (SEQ ID NO: 51) |
| RTSENIYSYLA | NAKTLPE | QHHYGTPPT |

Amino acid sequence of GT607 H chain variable region

| | | |
|---|---|---|
| CDR1 (SEQ ID NO: 52) | CDR2 (SEQ ID NO: 53) | CDR3 (SEQ ID NO: 54) |
| SYGMS | SVGNGGSYRYYPENLKG | RGAFPYFDV |

Amino acid sequence of GT607 L chain variable region

| | | |
|---|---|---|
| CDR1 (SEQ ID NO: 55) | CDR2 (SEQ ID NO: 56) | CDR3 (SEQ ID NO: 57) |
| SASSSVTYMH | ETSKLAS | QQWSSNPLT |

Amino acid sequence of GT165 H chain variable region

| | | |
|---|---|---|
| CDR1 (SEQ ID NO: 58) | CDR2 (SEQ ID NO: 59) | CDR3 (SEQ ID NO: 60) |
| NHHIN | YINPYNDYTNYNQKFKG | SDPAWFAY |

Amino acid sequence of GT165 L chain variable region (SEQ ID NO: 53)

| | | |
|---|---|---|
| CDR1 (SEQ ID NO: 61) | CDR2 (SEQ ID NO: 62) | |
| KASQDINKNIA | YTYTLQP | |

Amino acid sequence of GT114 H chain variable region

| | | |
|---|---|---|
| CDR1 (SEQ ID NO: 64) | CDR2 (SEQ ID NO: 65) | CDR3 (SEQ ID NO: 66) |
| SDSAWN | YIMYSGITSYNPSLKS | GYWYFDV |

Amino acid sequence of GT1 14L chain variable region (SEQ ID NO: 51)

| | | |
|---|---|---|
| CDR1 (SEQ ID NO: 67) | CDR2 (SEQ ID NO: 68) | CDR3 (SEQ ID NO: 69) |
| SASSSVSYMH | STSILAS | LQWITYRT |

For the assay of soluble GPC3 protein according to the present invention, antibodies having heavy chain and light chain variable regions having amino acid sequences with high homology or identity to the amino acid sequences of the heavy chain and light chain variable regions, respectively, of the antibodies described above can be used instead of the antibodies described above. The homology to the amino acid sequences of the heavy chain and light chain variable regions of the antibodies described above is at least 80% or higher, more preferably, for example, 90%, further preferably 95%, particularly preferably 98% or higher. The homology or the identity (similarity) can be calculated using any well-known algorithm, for example, Needleman-Wunsch, Smith-Waterman, BLAST, or FASTA and is calculated using, for example, the BLAST program (Atschul et al., J. Molec. Biol., 1990; 215: 403-410) under default conditions.

### Antibody recognizing same epitopes

A combination of antibodies respectively binding to the same epitopes as those for the antibodies described above can also be used as the "two different antibodies" in the method of the present invention. The antibody binding to the same epitope as that for each antibody can be obtained by using a method known in the art such as competitive ELISA or by assaying competition (cross-reactivity) with each antibody described above for the soluble GPC3 protein or an epitope fragment recognized by this antibody.

### Recombinant antibody

A combination of antibodies each having heavy chain and light chain variable regions identical to those of the GT30 antibody, the GT607 antibody, the GT165 antibody, or the GT114 antibody, or a combination of antibodies each having heavy chain CDR (CDR1, CDR2, and CDR3) and light chain CDR (CDR1, CDR2, and CDR3) regions identical to those of any of these antibodies can also be used as the "two different antibodies" of the present invention. A combination of chimeric antibodies, humanized antibodies, or human antibodies of these antibodies can also be used as the "two different antibodies" in the method of the present invention.

Each antibody having heavy chain and light chain variable regions identical to those of the GT30 antibody, the GT607 antibody, the GT165 antibody, or the GT114 antibody, or each antibody having heavy chain CDRs and light chain CDRs identical to those of any of these antibodies can be prepared by a recombination technique. Specifically, DNAs encoding the heavy chain and light chain variable regions of the anti-GPC3 N-terminal peptide antibody or the anti-GPC3 C-terminal peptide antibody of interest are incorporated into expression vectors having DNAs encoding desired antibody constant regions (C regions), and host cells are transformed with the resulting expression vectors and allowed to express antibodies.

For the antibody gene expression, the antibody heavy chain (H chain)- and light chain (L chain)-encoding DNAs can be separately incorporated into different expression vectors, with which a host cell can be co-transfected. Alternatively, the H chain- and L chain-encoding DNAs may be incorporated into a single expression vector, with which a host cell can be transformed (see WO 94/11523).

In addition to the host cells, transgenic animals can be used for the recombinant antibody production. For example, the antibody gene is inserted to a midpoint in a gene encoding a protein (goat β casein, etc.) specifically produced in milk to prepare a fusion gene. A DNA fragment containing the fusion gene having the antibody gene insert is injected into a goat embryo, which is in turn introduced into a female goat. The desired antibody is obtained from milk produced by transgenic goats (or progeny thereof) brought forth by the goat that has received the embryo. In addition, hormone may be appropriately used for the transgenic goats in order to increase the amount of milk containing the desired antibody produced from the transgenic goats (Ebert, K. M. et al., Bio/Technology (1994) 12, 699-702).

In the present invention, genetically recombinant antibodies that have been engineered artificially, for example, chimeric antibodies (humanized antibodies, etc.) can be used, in addition to the antibodies described above. These engineered antibodies can be produced by use of a known method.

The chimeric antibodies are obtained by linking the antibody V region-encoding DNAs obtained as described above to human antibody C region-encoding DNAs and incorporating the resulting products into expression vectors, which are then transferred to hosts to produce the chimeric antibodies. Chimeric antibodies useful for the present invention can be obtained by this known method.

The humanized antibodies, also called reshaped human antibodies, comprise complementarity determining regions (CDRs) of a non-human animal (e.g., mouse) antibody grafted in human antibodies, and a general genetic recombination method therefor is also known (see European Patent Application Publication No. EP 125023 and WO 96/02576).

The assay method of the present invention is an invention useful for a method for examining the possibility of liver cancer in a test subject by detecting solubilized GPC3 protein in a test sample isolated from the test subject, as with an invention described in, for example, Japanese Patent No. 4283227. Also, the assay method of the present invention is an invention also useful for a drug for predicting or determining recurrence after treatment of liver cancer, containing an anti-GPC3 antibody, as with an invention described in Japanese Patent No. 4658926. The assay method of the present invention can be used in a diagnosis method and a method for predicting or determining recurrence, comprising the assay method of the present invention. As shown later in Examples, the assay method of the present invention is capable of assaying soluble GPC3 protein contained in healthy subject blood on the order of several pg/mL and thus, is a more useful technique for early detection and prognosis of liver cancer as compared with the inventions related to the two patents described above.

More specifically, since the concentration range of soluble GPC3 in healthy subjects is approximately 15 to 174 pg/mL, soluble GPC3 detected at a level exceeding this range indicates the possibility that liver cancer has developed or recurred, and suggests that the risk can be reduced by early detection.

Also disclosed is an assay kit for use in the method for assaying soluble GPC3 protein, comprising the two different antibodies binding to different epitopes. These antibodies may be provided in a state immobilized on the carrier mentioned above or may be provided independently of the carrier. The kit may additionally comprise standard solutions of serially diluted soluble GPC3. Assay principles, etc., for use in the immunological assay kit are the same as in the immunological method mentioned above. In the assay kit, any of various aqueous solvents may be used as a solvent. Examples of the aqueous solvents include purified water, saline, and various buffers such as tris buffers, phosphate buffers, and phosphate-buffered saline. The pH of this buffer can be appropriately selected from among suitable pHs. The pH value used is not particularly limited and is generally selected within the range of pH 3 to 12.

The assay kit may appropriately contain, in addition to the components mentioned above, one or two or more components selected from proteins (e.g., bovine serum albumin (BSA), human serum albumin (HSA), casein, and salts thereof), various salts, various sugars, skimmed milk, various animal sera (e.g., normal rabbit serum), various antiseptics (e.g., sodium azide and antibiotics), activators, reaction accelerants, sensitivity-increasing substances (e.g., polyethylene glycol), nonspecific reaction-inhibiting substances, various surfactants (e.g., nonionic surfactants, amphoteric surfactants, and anionic surfactants), and the like. The concentrations of these components contained in the assay reagent are not particularly limited and are preferably 0.001 to 10% (W/V). Particularly preferred concentrations are appropriately selected within the range of 0.01 to 5% (W/V).

The assay kit may be further combined with other reagents, in addition to the components mentioned above. Examples of these other reagents include buffers, diluting solutions for biological samples, reagent diluting solutions, reagents containing labeling materials, reagents containing substances that generate signals such as color, reagents containing substances involved in the generation of signals such as color, reagents containing substances for calibration, and reagents containing substances for accuracy control.

The assay kit is not particularly limited by its form and may be provided as an integral-type assay kit comprising all of the components constituting the assay kit in order to carry out assay conveniently in a short time. Examples of the integral-type assay kit include ELISA kits, fluorescence immunoassay kits, and immunochromatography kits. The ELISA kit form comprises, for example, a first antibody-immobilized microplate, standard solutions of serially diluted soluble GPC3, a second antibody modified with an enzyme such as HRP, a washing buffer, and a substrate solution for the enzymatic reaction. The fluorescence immunoassay kit comprises, for example, a first antibody-immobilized optical waveguide, standard solutions of serially diluted soluble GPC3, a second antibody modified with a fluorescent material, and a washing buffer. The immunochromatography kit comprises a membrane housed in a reaction cassette where the first antibody is immobilized at one end (downstream) of the membrane. In an exemplary embodiment, a developing solution is placed at the other end (upstream) of the membrane; a pad supplemented with a substrate for the labeling agent is disposed in proximity (downstream) to the developing solution; and a pad supplemented with the second antibody labeled as described above is disposed in the central part of the membrane.

### Examples

Hereinafter, the present invention will be described in detail with reference to Examples. However, the present invention is not intended to be limited by these Examples.

### [Example 1]

A Balb/c or MRL/lpr mice were immunized a total of 6 to 9 times with GPC3 core (sGPC3-His) protein prepared by the C-terminal His tagging of the extracellular domain (positions 25 to 563) of GPC3 core variant derived from human glypican-3 (GPC3; SEQ ID NO: 70) by the exchange of serine at two heparan sulfate-binding sites (positions 495 and 509) to alanine. 3 days after the final immunization, the splenocytes of this mouse were fused with mouse myeloma cells SP2/0 by a routine method using PEG1500 or by using HVJ-E (Ishihara Sangyo Kaisha, Ltd.).

Next, hybridoma cells were screened by ELISA using sGPC3-His directly immobilized on a solid phase, ELISA using sGPC3-His bound with an anti-His antibody immobilized on a solid phase, or Cell ELISA using CHO cells stably expressing GPC3 (see WO 2006/006693).

### [Example 2]

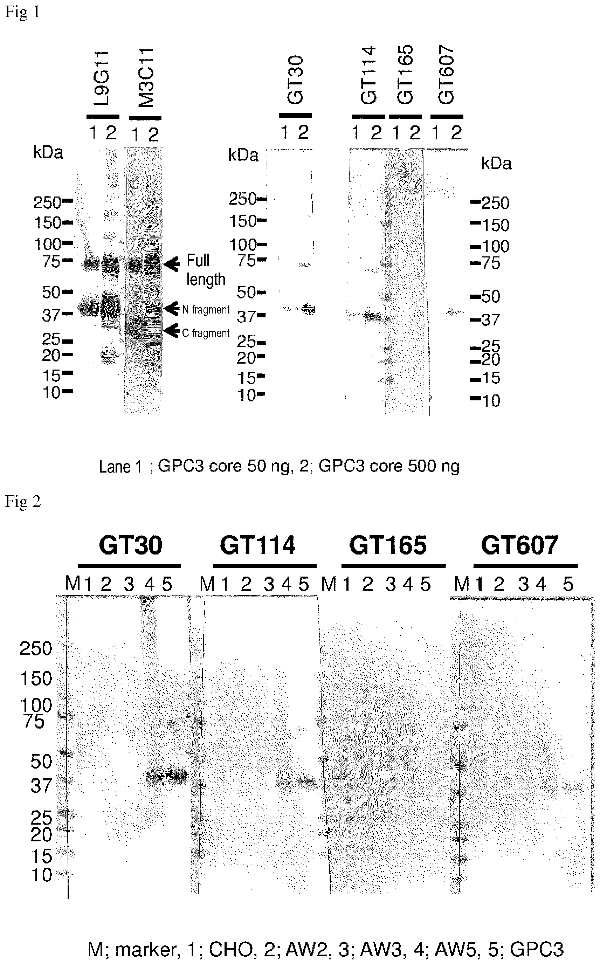

The sGPC3-His protein has been found to yield bands of approximately 86 kDa, approximately 43 kDa, and approximately 33 kDa, which correspond to a full length, a protease-cleaved N-terminal fragment, and a protease-cleaved C-terminal fragment, respectively, in SDS-PAGE under reductive conditions (WO 2006/006693). Mouse antibodies thus obtained by the screening were used in Western blot for sGPC3-His to select antibodies that did not recognize the C-terminal fragment. As a result, GT30, GT114, and GT607 as N-terminal fragment-recognizing antibodies, and GT165 whose recognition site was unidentified were obtained (Figure 1).

Subsequently, C-terminally His-tagged recombinant products of positions 1 to 196 (AW2), positions 1 to 218 (AW3), and positions 1 to 357 (AW5) of GPC3 were each expressed in CHO cells, and pellets of the CHO cells expressing each recombinant product were used in Western blot. As a result, as shown in Figure 2 and Table 1, GT30, GT114, and GT607 were confirmed to have reactivity with AW5 or sGPC3-His, but no reactivity with AW2 and AW3, indicating that these antibodies bind to positions 219 to 357 of GPC3. On the other hand, GT165 was confirmed to have reactivity with none of these products, suggesting the possibility that this antibody strongly recognizes conformation.

**[Table 1]**

| Clone | Immunized mouse | Isotype | Western blot analysis | | | |
|---|---|---|---|---|---|---|
| | | | sGPC3-His | AW2 (1-196) | AW3 (1-218) | AW5 (1-357) |
| GT114 | BALB/c | IgG1 | ++ | - | - | + |
| GT607 | BALB/c | IgG1 | ++ | - | - | + |
| GT30 | MRL | IgG1 | ++ | - | - | ++ |
| GT165 | MRL | IgG1 | +/- | - | - | - |

### [Example 3]

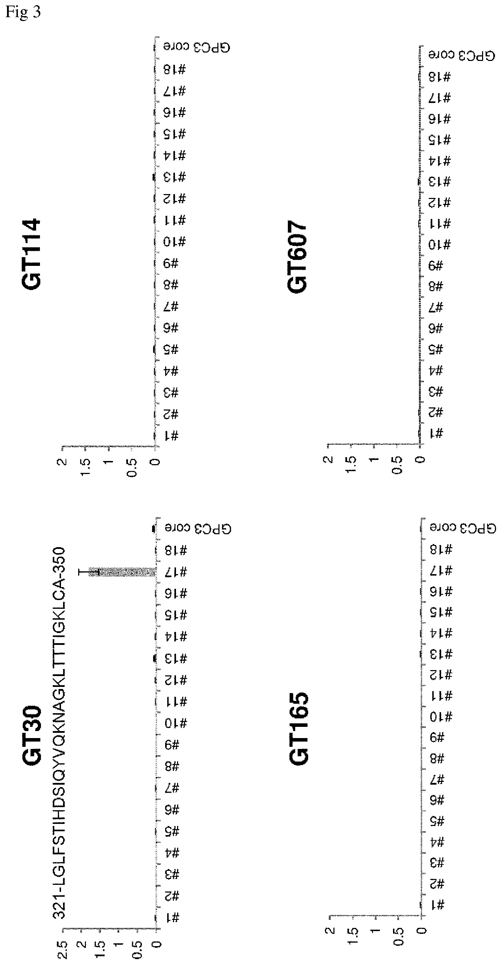

In order to identify more detailed epitopes for the antibodies, the amino acid sequence of GPC3 was divided into peptides of 30 residues containing 10-residue overlaps, and the corresponding peptides were synthesized (SEQ ID NOs: 1 to 29 in Table 2). Each peptide thus synthesized was dissolved at a concentration of 1 mg/mL in dimethyl sulfoxide and then diluted to 1 µg/mL with PBS. The diluted peptide solution was added at 70 µL/well to a 96-well plate and immobilized thereon at room temperature for 1 hour. After removal of unimmobilized peptides, 20% Blocking-one (Nacalai Tesque, Inc.) was added thereto as a blocking buffer solution at 200 µL/well, and the plate was blocked overnight. After removal of the blocking buffer solution, each well was washed with TBS-T three times, and each antibody diluted to 2 µg/mL with a blocking buffer was then added thereto at 70 µL/well. One hour later, the antibody was removed, and each well was washed three times by the addition of TBS-T at 200 µL/well. An HRP-bound anti-mouse antibody was added at 70 µL/well to each well and reacted therewith for 1 hour. After removal of the antibody, each well was washed with 200 µL/well of TBS-T three times, and a chromogenic reagent ABTS Peroxidase Substrate System (1 component, KPL, Kirkegaard & Perry Laboratories, Inc.) was then added thereto at 70 µL/well and reacted at room temperature for 15 to 30 to develop color. The reaction was terminated by the addition of 1% SDS at 70 µL/well, and the absorbance at 405 nm was measured using a plate reader. As a result, as shown in Figure 3, GT30 was found to bind to the peptide of SEQ ID NO: 17, whereas the specific binding of GT114, GT165, and GT607 was not found for these peptides.

These results suggest that GT30 binds to a site from position 321 to position 350 of GPC3 and also suggest the possibility that GT165 as well as GT114 and GT607 recognize conformation.

**[Table 2]**

| Amino acid position | Sequence | SEQ ID NO |
|---|---|---|
| 1-30 | MAGTVRTACLWAMLLSLDFPGQAQPPPPP | 1 |
| 21-50 | PGQAQPPPPPPDATCHQVRSFFQRLQPGLK | 2 |
| 41-70 | FFQRLQPGLKWVPETPVPGSDLQVCLPKGP | 3 |
| 61-90 | DLQVCLPKGPTCCSRKMEEKYQLTARLNME | 4 |
| 81-110 | YQLTARLNMEQLLQSASMELKFLIIQNAAV | 5 |
| 101-130 | KFLIIQNAAVFQEAFEIVVRHAKNYTNAMF | 6 |
| 121-150 | HAKNYTNAMFKNNYPSLTPQAFEFVGEFFT | 7 |
| 141-170 | AFEFVGEFFTDVSLYILGSDINVDDMVNEL | 8 |
| 161-190 | INVDDMVNELFDSLFPVIYTQLMNPGLPDS | 9 |
| 181-210 | QLMNPGLPDSALDINECLRGARRDLKVFGN | 10 |
| 201-230 | ARRDLKVFGNFPKLIMTQVSKSLQVTRIFL | 11 |
| 221-250 | KSLQVTRIFLQALNLGIEVINTTDHLKFSK | 12 |
| 241-270 | NTTDHLKFSKDCGRMLTRMWYCSYCQGLMM | 13 |
| 261-290 | YCSYCQGLMMVKPCGGYCNVVMQGCMAGVV | 14 |
| 281-310 | VMQGCMAGWEIDKYWREYILSLEELVNGM | 15 |
| 301-330 | LSLEELVNGMYRIYDMENVLLGLFSTIHDS | 16 |
| 321-350 | LGLFSTIHDSIQYVQKNAGKLTTTIGKLCA | 17 |
| 341-370 | LTTTIGKLCAHSQQRQYR^SAYYPEDLFIDK | 18 |
| 361-390 | YYPEDLFIDKKVLKVAHVEHEETLSSRRRE | 19 |
| 381-410 | EETLSSRRRELIQKLKSFISFYSALPGYIC | 20 |
| 401-430 | FYSALPGYICSHSPVAENDTLCWNGQELVE | 21 |
| 421-450 | LCWNGQELVERYSQKAARNGMKNQFNLHEL | 22 |
| 441-470 | MKNQFNLHELKMKGPEPVVSQIIDKLKHIN | 23 |
| 461-490 | QIIDKLKHINQLLRTMSMPKGRVLDKNLDE | 24 |
| 481-510 | GRVLDKNLDEEGFESGDCGDDEDECIGGSG | 25 |
| 501-530 | DEDECIGGSGDGMIKVKNQLRFLAELAYDL | 26 |
| 521-550 | RFLAELAYDLDVDDAPGNSQQATPKDNEIS | 27 |
| 541-570 | QATPKDNEISTFHNLGNVHSPLKLLTSMAI | 28 |
| 551-580 | TFHNLGNVHSPLKLLTSMAISVVCFFFLVH | 29 |

### [Example 4]

Next, in order to analyze the binding competition of each antibody recognizing the GPC3 N-terminal fragment, each antibody was used in a binding competition test.

Specifically, a 1 µg/mL sGPC3 solution (in PBS) was added at 50 µL/well to a 96-well transparent microplate (MaxiSoap manufactured by Thermo Fisher Scientific Inc.), and the microplate was left standing overnight at room temperature. Subsequently, the microplate was washed with a washing solution (TBS containing 0.01% Triton X-100) once. Then, a blocking solution (Blocking-One manufactured by Nacalai Tesque, Inc.) was added thereto at 200 µL/well, and the microplate was left standing at room temperature for 1 hour. The microplate was washed with a washing solution three times. Then, a 50 µg/mL unlabeled anti-GPC3 antibody solution (in PBS containing 20% Blocking-One) was added thereto at 25 µL/well, and the mixture was stirred at room temperature for 1 minute. A 0.5 µg/mL biotin-labeled anti-GPC3 antibody solution (in PBS containing 20% Blocking-One) was further added thereto at 25 µL/well, and the mixture was stirred at room temperature for 1 hour. Subsequently, the microplate was washed with a washing solution three times. Then, a solution of StreptAvidin-HRP (manufactured by Prozyme) diluted 10,000-fold with a diluting solution (TBS containing 10% Block Ace and 0.1% Tween 20) was added thereto at 50 µL/well, and the mixture was stirred at room temperature for 30 minutes. The microplate was washed with a washing solution five times. Then, a chromogenic substrate solution (1-Step Turbo TMB manufactured by Thermo Fisher Scientific Inc.) was added thereto at 50 µL/well, and the absorbance at 450 nm (O.D. 450) was measured using a microplate reader.

**[Table 3]**

| - | | | Labeled antibody | | | |
|---|---|---|---|---|---|---|
| | | | GT30 | GT114 | GT607 | GT1 65 |
| O.D. 450 | Unlabeled antibody (added in 100-fold amount) | | 1.840 | 2.299 | 2.181 | 1 .678 |
| | | GT30 | 0.070 | 2.315 | 2.125 | 0.098 |
| | | GT11 4 | 2.140 | 0.251 | 0.307 | 1.818 |
| | | GT607 | 2.064 | 1.132 | 0.135 | 0.129 |
| | | GT165 | 0.846 | 2.280 | 1.248 | 0.111 |
| Inhibitory effect (%) | | GT30 | 96% | 0%> | 3% | 94% |
| Unlabeled antibody (added in 100-fold amount) | | GT114 | 0%> | 89% | 86% | 0%> |
| | | GT607 | 0%> | 51% | 94% | 92% |
| | | GT165 | 54% | 1% | 43% | 93% |

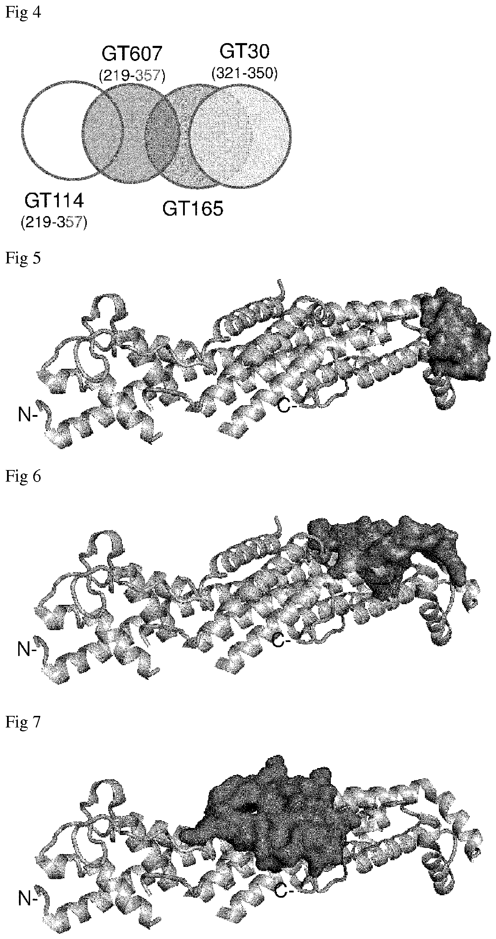

As shown in Table 3, GT30 and GT165 mutually inhibited their binding to GPC3. Therefore, the epitopes for GT165 and GT30 seem to be located in proximity. Although GT114 and GT607 were free from inhibition by GT30, their binding to GPC3 was inhibited in the coexistence of GT114 and GT607. Therefore, the epitopes for GT114 and GT607 seem to be located in proximity. On the other hand, GT114 was also free from inhibition by GT165, whereas GT607 underwent the inhibition of binding by GT165. Therefore, the binding site of GT607 was found to not overlap with the binding site of GT30, but to be located near the binding region of GT165 (Figure 4).

### [Example 5]

The results described above suggested that GT114, GT165, and GT607 recognize conformation, and, particularly, GT165 binds to near the site from position 321 to position 350, which is an epitope region for GT30. Therefore, a possible binding region was identified using a conformational model of human GPC3.

First, on the hypothesis that the structure of fruit fly Dally-like protein (PDB ID: 3ODN; Kim M.S. et al., Proc Natl Acad Sci USA 2011; 108: 13112-13117) having high homology to human GPC3 and having reported results of X-ray crystallography would be the conformation of human GPC3 (containing a proline residue at position 29 to a lysine residue at position 486 except for tyrosine at position 357 to valine at position 372), the interaction of each antibody was predicted using PyMOL Molecular Graphics System, Version 1.5.0.4 (manufactured by Schrodinger LLC). The conformation of the glypican molecule has been reported to be well conserved across species (Svensson G. et al., J Biol Chem 2012; 287: 14040-14051).

As a result, the epitope region for GT30 from position 321 to position 350 was presumed to have an α-helix structure. In order to analyze a binding mode for this region, three mouse monoclonal antibody Fabs (PDB IDs: 2CMR, 2XRA, and 3V6Z) found to recognize an α-helix structure and having reported results of X-ray crystallography were used as helix structure recognition models. The X-ray crystal structure of a mouse IgG1 antibody (PDB ID: 1IGY) was further overlaid with each of these three Fabs. As a result, a total of 12 possible binding modes including all combinations of two types of helix-binding sites for the region from position 321 to position 350, the binding modes of 3 types of Fabs (2CMR, 2XRA, and 3V6Z), and two mouse IgG1 Fabs were predicted for mouse IgG1 antibodies recognizing two helix moieties in the region from position 321 to position 350 of human GPC3.

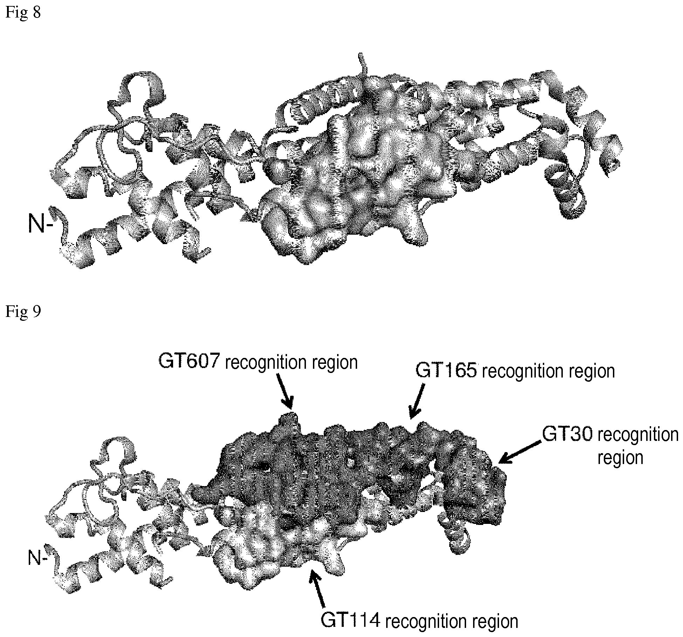

For each of these 12 IgG1 binding models, a region on human GPC3 that came in contact with Fab within 4 angstroms was identified as a binding region for GT165, which is an antibody competing with GT30 for antigen binding. Likewise, a region capable of competing with the binding region for GT165 was identified as a binding region for GT607. Subsequently, on the basis of the binding region for GT607, a region capable of competing therewith was identified as a binding region for GT114. From these results, the results of Examples 2 and 3, and the results about competitive relationships shown in Table 3 and Figure 4, regions shown in Figures 5 to 9 were identified as binding regions for GT30, GT165, GT607, and GT114. Of these recognition regions identified here, human GPC3 amino acid residues that are important for the antigen binding of each antibody are described in Table 4, in light of the results of Examples 2 and 3. All human GPC3 amino acid residues that may serve as recognition regions of each antibody predicted from the structural models are shown in Table 5.

**[Table 4]**

| GT30 | GT165 | | GT607 | GT114 |
|---|---|---|---|---|
| N337 | A128 | G322 | K221 | S220 |
| G339 | M129 | S325 | R297 | K221 |
| K340 | K131 | T326 | E298 | I228 |
| L341 | N132 | H328 | I300 | Q231 |
| T343 | N133 | D329 | L301 | A232 |
| T344 | Y134 | S330 | S302 | L235 |
| G316 | P135 | Q332 | E301 | E291 |
| K347 | F171 | Y333 | E305 | K294 |
| L348 | F208 | Q335 | L306 | Y295 |
| C349 | G209 | K336 | N308 | E298 |
| A350 | N210 | N337 | G309 | L301 |
| | F211 | A338 | Y311 | S302 |
| | P212 | G339 | R312 | E305 |
| | L214 | K340 | I313 | N308 |
| | I215 | T344 | Y314 | G309 |
| | Q218 | | D315 | |
| | N318 | | N318 | |

**[Table 5]**

| Human GPC3 amino acid position | Human GPC3 amino acid residue |
|---|---|
| 129 | Met |
| 133 | Asn |
| 134 | Tyr |
| 182 | Leu |
| 186 | Gly |
| 194 | Ile |
| 205 | Leu |
| 208 | Phe |
| 209 | Gly |
| 210 | Asn |
| 211 | Phe |
| 214 | Leu |
| 215 | Ile |
| 218 | Gin |
| 318 | Asn |
| 351 | His |

### [Example 6]

### (Construction of ELISA assay system using magnetic particle)

On the basis of the results of Example 4, two types shown in Table 6 were selected as combinations of antibodies capable of constructing soluble GPC3 assay systems.

**[Table 6]**

| Assay system No. | On solid-phase side | On label side |
|---|---|---|
| 1 | GT114 | GT165 |
| 2 | GT30 | GT607 |

For each assay system shown in Table 6, the antibody on a slid-phase side was immobilized on a magnetic particle (Magnosphere MS300/Carboxyl manufactured by JSR Life Sciences Corp.), while the antibody on a label side was labeled with alkaline phosphatase in order to achieve high sensitivity.

### [Example 7]

### (Assay of soluble GPC3 by chemiluminescent enzyme immunoassay using magnetic particle)

The soluble GPC3 was assayed by chemiluminescent enzyme immunoassay using magnetic particles according to the following method:

0.06% (w/v) GT30 or GT114 antibody-bound magnetic particles were added at 25 µL/wee to a 96-well white microplate (manufactured by Corning Inc.). Subsequently, a solution sample containing soluble GPC3 was added thereto at 25 µL/well. Each solution of the GT607 or GT165 antibody labeled with alkaline phosphatase was further added thereto at 25 µL/well. Then, the 96-well white microplate was stirred at 25°C for 20 minutes. The antibody-bound magnetic particles were washed with a washing solution (TBS containing 0.01% Triton X-100) five times while magnetism was collected with a microplate washer (HydroFlex manufactured by Tecan Trading AG). A luminescent substrate solution (Lumipulse substrate solution manufactured by FUJIREBIO Inc.) was further added thereto at 50 µL/well. Five minutes later, the luminescence intensity was measured using a chemiluminescence detector (GloMax 96 manufactured by Promega Corp.).

### [Example 8]

### (Detection limit and quantification limit)

For the detection limit, soluble GPC3 solution samples prepared at 0 to 10 pg/mL were each assayed 10 times. A mean and standard deviation (SD) of the luminescence intensity values measured at each concentration were calculated. Then, the lowest concentration at which the range of the mean measured luminescence intensity value ± 3SD did not overlap with the range of the mean measured luminescence intensity at the concentration 0 pg/mL ± 3SD was defined as the detection limit.

For the quantification limit, soluble GPC3 solution samples prepared at 0 to 10 pg/mL were each assayed 10 times. The concentration at which CV of the measurement values calculated from a calibration curve was 10% or less was defined as the quantification limit.

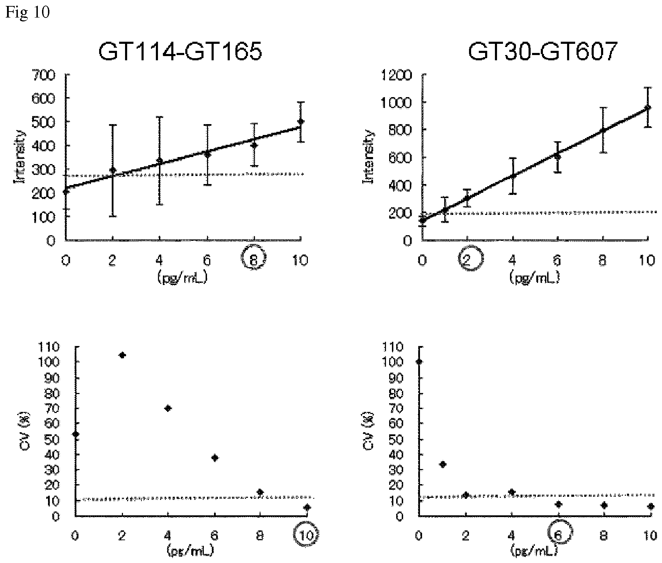

As a result, as shown in Figure 10, all of the constructed assay systems had a detection limit and a quantification limit of 10 pg/mL or lower and were confirmed to be capable of assaying soluble GPC3 highly sensitively.

### [Example 9]

### (Dilution linearity test of liver cancer specimen)

A calibration curve was prepared from soluble GPC3 solutions each prepared at 0, 10, 25, 50, 100, 250, 500, or 1000 pg/mL. Soluble GPC3 in serially diluted liver cancer specimens was assayed by the same procedures as in Example 7.

As a result, as shown in Table 7, values were calculated by multiplying each measurement value by each dilution ratio and evaluated for dilution linearity. Consequently, the dilution linearity was confirmed.

**[Table 7]**

| | Dilution ratio | GT114-GT165 | GT30-GT607 |
|---|---|---|---|
| Mean measurement value **(pg/mL)** | **1** | **773** | **763** |
| | **2** | **395** | **370** |
| | **4** | **192** | **181** |
| | **8** | **121** | **92** |
| | **16** | **52** | **53** |
| Measurement value × dilution ratio **(pg/mL)** | **1** | **773** | **763** |
| | **2** | **790** | **740** |
| | **4** | **768** | **724** |
| | **8** | **968** | **736** |
| | **16** | **832** | **848** |
| Linearity evaluation **(%)** | **1** | **100** | **100** |
| | **2** | **102** | **97** |
| | **4** | **99** | **95** |
| | **8** | **125** | **96** |
| | **16** | **108** | **111** |

### [Example 10]

### (Assay using healthy subjects' specimens)

A calibration curve was prepared from soluble GPC3 solutions each prepared at 0, 10, 25, 50, 100, 250, 500, or 1000 pg/mL. Soluble GPC3 in healthy subject sera was assayed by the same procedures as in Example 7. 21 healthy subjects' sera were used in the assay, and a mean of the measurement values was determined.

**[Table 8]**

| | | GT114-GT165 | GT30-GT607 |
|---|---|---|---|
| Measurement value (pg/mL) | Normal human 1 | 32 | 103 |
| | Normal human 2 | 53 | 99 |
| | Normal human 3 | 32 | 107 |
| | Normal human 4 | 15 | 109 |
| | Normal human 5 | 35 | 171 |
| | Normal human 6 | 21 | 129 |
| | Normal human 7 | 55 | 119 |
| | Normal human 8 | 20 | 138 |
| | Normal human 9 | 58 | 174 |
| | Normal human 10 | 25 | 86 |
| | Normal human 11 | 21 | 66 |
| | Normal human 12 | 41 | 125 |
| | Normal human 13 | 64 | 165 |
| | Normal human 14 | 55 | 122 |
| | Normal human 15 | 16 | 47 |
| | Normal human 16 | 17 | 128 |
| | Normal human 17 | 73 | 142 |
| | Normal human 18 | 52 | 127 |
| | Normal human 19 | 16 | 88 |
| | Normal human 20 | 51 | 116 |
| | Normal human 21 | 31 | 104 |
| | Mean | 37 | 117 |

As a result, as shown in Table 8, all of the measurement values of the soluble GPC3 in the healthy subjects' sera were above the detection limit and the quantification limit of each assay system, and the concentration range of the soluble GPC3 was 15 to 174 pg/mL. From these results, all of the constructed assay systems were confirmed to be capable of detecting soluble GPC3 contained at as trace as several tens to several hundreds of pg/mL in healthy subject serum.

### [Example 11]

### Sequence analysis of obtained antibody

Total RNA was extracted from each of the hybridomas producing the antibodies GT30, GT114, GT165, GT607, and GT30 obtained as described above, and cDNA was synthesized therefrom using reverse transcriptase. Then, the mouse antibody genes were amplified by PCR, and nucleotide sequences each encoding the amino terminus to the variable region were determined (SEQ ID NOs: 30 to 37). The amino acid sequences of the variable regions are shown in SEQ ID NOs: 38 to 45. The sequences of their CDR regions are shown in SEQ ID NOs: 46 to 69.

### [Example 12]

In order to confirm the efficacy and safety of GC33 in advanced and/or recurrent hepatocellular carcinoma (HCC) patients, a multicenter, randomized, double-blind, placebo-controlled phase-II clinical trial involving administering 1600 mg of GC33 every other week was conducted targeting previously treated human adult patients with unresectable advanced or metastatic hepatocellular carcinoma (NP27884 study). GC33 is a genetically recombinant humanized IgG1 monoclonal antibody binding to human GPC3 with high affinity (WO2006/006693).

The concentration of soluble GPC3 in serum before the first dose for the cases given GC33 or a placebo in the GPC3-targeting treatment was confirmed to be able to be assayed with a combination of two different antibodies binding to soluble GPC3 (combination of the GT30 antibody and the GT607 antibody or a combination of GT114 and GT165).

An antibody-bound particle solution containing GT30 or GT114 bound with magnetic particles (MS300 manufactured by JSR Life Sciences Corp.) was added at 25 µL/well to a 96-well microplate. Subsequently, a standard sample solution for a calibration curve or an appropriately diluted serum sample was added thereto at 25 µL/well, and alkaline phosphatase-labeled GT607 or GT165 was further added thereto at 25 µL/well. After shaking at 25°C for 20 minutes, each well was washed with a washing solution five times with magnetism collected using Dyna-Mag-96 Side Skirted (manufactured by VERITAS Corp.). A luminescent substrate solution preheated to 37°C was added thereto at 50 µL/well. The plate was shaken at room temperature for 1 minute and then left standing for 4 minutes to develop light. The chemiluminescence intensity was measured using a luminometer (manufactured by VERITAS Corp.). The GPC3 standard used in the standard sample solution for a calibration curve was recombinant GPC3 with serine residues at positions 495 and 509 substituted by alanine residues so as to prevent the binding of a heparan sulfate sugar chain (Hippo et al., Cancer Res. (2004) 64, 2418-2423).

The calibration curve (standard curve) prepared on the basis of standard samples containing the recombinant GPC3 was used to calculate the GPC3 antigen in the serum of each patient from the obtained chemiluminescence intensity of each well.

### Industrial Applicability

The present invention is useful for early detection at an initial stage in development, selection of an anticancer agent used, and post-treatment prognosis for cancer, particularly, liver cancer.

## Claims

1. A method for assaying soluble GPC3 protein in a test sample, comprising using two different antibodies binding to different epitopes contained in an amino acid sequence from position 128 to position 357 of GPC3 protein represented by SEQ ID NO: 70,
wherein the different epitopes are an epitope that is bound by an antibody having a heavy chain variable region comprising a sequence having 80% or higher homology to the sequence represented by SEQ ID NO: 38 and a light chain variable region comprising a sequence having 80% or higher homology to the sequence represented by SEQ ID NO: 39, and an epitope that is bound by an antibody having a heavy chain variable region comprising a sequence having 80% or higher homology to the sequence represented by SEQ ID NO: 40 and a light chain variable region comprising a sequence having 80% or higher homology to the sequence represented by SEQ ID NO: 41, or
an epitope that is bound by an antibody having a heavy chain variable region comprising a sequence having 80% or higher homology to the sequence represented by SEQ ID NO: 44 and a light chain variable region comprising a sequence having 80% or higher homology to the sequence represented by SEQ ID NO: 45, and an epitope that is bound by an antibody having a heavy chain variable region comprising a sequence having 80% or higher homology to the sequence represented by SEQ ID NO: 42 and a light chain variable region comprising a sequence having 80% or higher homology to the sequence represented by SEQ ID NO: 43.

2. The method according to claim 1, wherein the two different antibodies are a combination of an antibody having CDR regions identical to CDR regions contained in a heavy chain variable region shown in SEQ ID NO: 38 and CDR regions contained in a light chain variable region shown in SEQ ID NO: 39, and an antibody having CDR regions identical to CDR regions contained in a heavy chain variable region shown in SEQ ID NO: 40 and CDR regions contained in a light chain variable region shown in SEQ ID NO: 41, or
a combination of an antibody having CDR regions identical to CDR regions contained in a heavy chain variable region shown in SEQ ID NO: 44 and CDR regions contained in a light chain variable region shown in SEQ ID NO: 45, and an antibody having CDR regions identical to CDR regions contained in a heavy chain variable region shown in SEQ ID NO: 42 and CDR regions contained in a light chain variable region shown in SEQ ID NO: 43.

3. The method according to claim 2, wherein the CDR regions are CDR1, CDR2, and CDR3 regions based on the Kabat numbering.

4. The method according to claim 1, wherein the two different antibodies are a combination of an antibody having a heavy chain variable region whose CDR1, CDR2, and CDR3 comprise the amino acid sequences represented by SEQ ID NOs: 46, 47, and 48, respectively, and having a light chain variable region whose CDR1, CDR2, and CDR3 comprise the amino acid sequences represented by SEQ ID NOs: 49, 50, and 51, respectively, and an antibody having a heavy chain variable region whose CDR1, CDR2, and CDR3 comprise the amino acid sequences represented by SEQ ID NOs: 52, 53, and 54, respectively, and having a light chain variable region whose CDR1, CDR2, and CDR3 comprise the amino acid sequences represented by SEQ ID NOs: 55, 56, and 57, respectively, or
a combination of an antibody having a heavy chain variable region whose CDR1, CDR2, and CDR3 comprise the amino acid sequences represented by SEQ ID NOs: 58, 59, and 60, respectively, and having a light chain variable region whose CDR1, CDR2, and CDR3 comprise the amino acid sequences represented by SEQ ID NOs: 61, 62, and 63, respectively, and an antibody having a heavy chain variable region whose CDR1, CDR2, and CDR3 comprise the amino acid sequences represented by SEQ ID NOs: 64, 65, and 66, respectively, and having a light chain variable region whose CDR1, CDR2, and CDR3 comprise the amino acid sequences represented by SEQ ID NOs: 67, 68, and 69, respectively.

5. The method according to claim 1, wherein the two different antibodies are a combination of an antibody having a heavy chain variable region shown in SEQ ID NO: 38 and a light chain variable region shown in SEQ ID NO: 39, and an antibody having a heavy chain variable region shown in SEQ ID NO: 40 and a light chain variable region shown in SEQ ID NO: 41, or
a combination of an antibody having a heavy chain variable region shown in SEQ ID NO: 44 and a light chain variable region shown in SEQ ID NO: 45, and an antibody having a heavy chain variable region shown in SEQ ID NO: 42 and a light chain variable region shown in SEQ ID NO: 43.

6. The method according to any one of claims 1 to 5, wherein any one of the two different antibodies is bound with a magnetic particle.

7. The method according to any one of claims 1 to 6, wherein the test sample is a tissue sample, a whole blood sample, a plasma sample, or a serum sample isolated from a human.

## Patentansprüche

1. Verfahren zum Testen von löslichem GPC3-Protein in einer Testprobe, umfassend die Verwendung von zwei verschiedenen Antikörpern, die an verschiedene Epitope binden, die in einer Aminosäuresequenz von Position 128 bis Position 357 des GPC3-Proteins, dargestellt durch SEQ ID NO: 70, enthalten sind,
wobei die verschiedenen Epitope ein Epitop sind, das durch einen Antikörper gebunden ist, der Folgendes aufweist: eine variable Region der schweren Kette, die eine Sequenz mit 80 % oder höherer Homologie zu der durch SEQ ID NO: 38 dargestellten Sequenz umfasst, und eine variable Region der leichten Kette, die eine Sequenz mit 80 % oder höherer Homologie zu der durch SEQ ID NO: 39 dargestellten Sequenz umfasst, und ein Epitop, das durch einen Antikörper gebunden ist, der Folgendes aufweist: eine variable Region der schweren Kette, die eine Sequenz mit 80 % oder höherer Homologie zu der durch SEQ ID NO: 40 dargestellten Sequenz umfasst, und eine variable Region der leichten Kette, die eine Sequenz mit 80 % oder höherer Homologie zu der durch SEQ ID NO: 41 dargestellten Sequenz umfasst, oder
ein Epitop, das durch einen Antikörper gebunden ist, der Folgendes aufweist: eine variable Region der schweren Kette, die eine Sequenz mit 80 % oder höherer Homologie zu der durch SEQ ID NO: 44 dargestellten Sequenz umfasst, und eine variable Region der leichten Kette, die eine Sequenz mit 80 % oder höherer Homologie zu der durch SEQ ID NO: 45 dargestellten Sequenz umfasst, und ein Epitop, das durch einen Antikörper gebunden ist, der Folgendes aufweist: eine variable Region der schweren Kette, die eine Sequenz mit 80 % oder höhere Homologie zu der durch SEQ ID NO: 42 dargestellten Sequenz umfasst, und eine variable Region der leichten Kette, die eine Sequenz mit 80 % oder höherer Homologie zu der durch SEQ ID NO: 43 dargestellten Sequenz umfasst.

2. Verfahren nach Anspruch 1, wobei die beiden unterschiedlichen Antikörper eine Kombination sind aus einem Antikörper mit CDR-Regionen, die identisch sind mit CDR-Regionen, die in einer in SEQ ID NO: 38 gezeigten variablen Region der schweren Kette enthalten sind, und CDR-Regionen, die in einer in SEQ ID NO: 39 gezeigten variablen Region der leichten Kette enthalten sind, und einem Antikörper mit CDR-Regionen, die identisch sind mit CDR-Regionen, die in einer in SEQ ID NO: 40 gezeigten variablen Region der schweren Kette enthalten sind, und CDR-Regionen, die in einer in SEQ ID NO: 41 gezeigten variablen Region der leichten Kette enthalten sind, oder
eine Kombination aus einem Antikörper mit CDR-Regionen, die identisch sind mit CDR-Regionen, die in einer in SEQ ID NO: 44 gezeigten variablen Region der schweren Kette enthalten sind, und CDR-Regionen, die in einer in SEQ ID NO: 45 gezeigten variablen Region der leichten Kette enthalten sind, und einem Antikörper mit CDR-Regionen, die identisch sind mit CDR-Regionen, die in einer in SEQ ID NO: 42 gezeigten variablen Region der schweren Kette enthalten sind, und CDR-Regionen, die in einer in SEQ ID NO: 43 gezeigten variablen Region der leichten Kette enthalten sind.

3. Verfahren nach Anspruch 2, wobei die CDR-Regionen CDR1-, CDR2- und CDR3-Regionen, beruhend auf der Kabat-Nummerierung, sind.

4. Verfahren nach Anspruch 1, wobei die zwei unterschiedlichen Antikörper eine Kombination sind aus einem Antikörper, der Folgendes aufweist: eine variable Region der schweren Kette, deren CDR1, CDR2 und CDR3 die durch die SEQ ID Nummern: 46, 47 bzw. 48 dargestellten Aminosäuresequenzen umfassen, und eine variable Region der leichten Kette, deren CDR1, CDR2 und CDR3 die durch die SEQ ID Nummern: 49, 50 bzw. 51 dargestellten Aminosäuresequenzen umfassen, und einem Antikörper, der Folgendes aufweist: eine variable Region der schweren Kette, deren CDR1, CDR2 und CDR3 die durch die SEQ ID Nummern: 52, 53 bzw. 54 dargestellten Aminosäuresequenzen umfassen, und eine variable Region der leichten Kette, deren CDR1, CDR2 und CDR3 die durch die SEQ ID Nummern: 55, 56 bzw. 57 dargestellten Aminosäuresequenzen umfassen, oder
eine Kombination aus einem Antikörper, der Folgendes aufweist: eine variable Region der schweren Kette, deren CDR1, CDR2 und CDR3 die durch die SEQ ID Nummern: 58, 59 bzw. 60 dargestellten Aminosäuresequenzen umfassen, und eine variable Region der leichten Kette, deren CDR1, CDR2 und CDR3 die durch die SEQ ID Nummern: 61, 62 bzw. 63 dargestellten Aminosäuresequenzen umfassen, und einem Antikörper, der Folgendes aufweist: eine variable Region der schweren Kette, deren CDR1, CDR2 und CDR3 die durch die SEQ ID Nummern: 64, 65 bzw. 66 dargestellten Aminosäuresequenzen umfassen, und eine variable Region der leichten Kette, deren CDR1, CDR2 und CDR3 die durch die SEQ ID Nummern: 67, 68 bzw. 69 dargestellten Aminosäuresequenzen umfassen.

5. Verfahren nach Anspruch 1, wobei die zwei verschiedenen Antikörper eine Kombination sind aus einem Antikörper, der Folgendes aufweist: eine in SEQ ID NO: 38 gezeigte variable Region der schweren Kette und eine in SEQ ID NO: 39 gezeigte variable Region der leichten Kette, und einem Antikörper, der Folgendes aufweist: eine in SEQ ID NO: 40 gezeigte variable Region der schweren Kette und eine in SEQ ID NO: 41 gezeigte variable Region der leichten Kette, oder
eine Kombination aus einem Antikörper, der Folgendes aufweist: eine in SEQ ID NO: 44 gezeigte variable Region der schweren Kette und eine in SEQ ID NO: 45 gezeigte variable Region der leichten Kette, und einem Antikörper, der Folgendes aufweist: eine in SEQ ID NO: 42 gezeigte variable Region der schweren Kette und eine in SEQ ID NO: 43 gezeigte variable Region der leichten Kette.

6. Verfahren nach einem der Ansprüche 1 bis 5, wobei einer der beiden verschiedene Antikörper mit einem magnetischen Teilchen gebunden ist.

7. Verfahren nach einem der Ansprüche 1 bis 6, wobei die Testprobe eine Gewebeprobe, eine Vollblutprobe, eine Plasmaprobe oder eine Serumprobe ist, die von einem Menschen isoliert wurde.

## Revendications

1. Procédé pour doser la protéine GPC3 soluble dans un échantillon à tester, comprenant l'utilisation de deux anticorps différents se liant à différents épitopes contenus dans une séquence d'acides aminés de la position 128 à la position 357 de la protéine GPC3 représentée par SEQ ID NO: 70,
dans lequel les différents épitopes sont un épitope qui est lié par un anticorps ayant une région variable de chaîne lourde comprenant une séquence ayant 80% ou plus d'homologie avec la séquence représentée par SEQ ID NO: 38 et une région variable de chaîne légère comprenant une séquence ayant 80% ou plus d'homologie avec la séquence représentée par SEQ ID NO: 39, et un épitope qui est lié par un anticorps ayant une région variable de chaîne lourde comprenant une séquence ayant 80% ou plus d'homologie avec la séquence représentée par SEQ ID NO: 40 et une région variable de chaîne légère comprenant une séquence ayant 80% ou plus d'homologie avec la séquence représentée par SEQ ID NO: 41, ou
un épitope qui est lié par un anticorps ayant une région variable de chaîne lourde comprenant une séquence ayant 80% ou plus d'homologie avec la séquence représentée par SEQ ID NO: 44 et une région variable de chaîne légère comprenant une séquence ayant 80% ou plus d'homologie avec la séquence représentée par SEQ ID NO: 45, et un épitope qui est lié par un anticorps ayant une région variable de chaîne lourde comprenant une séquence ayant 80% ou plus d'homologie avec la séquence représentée par SEQ ID NO: 42 et une région variable de chaîne légère comprenant une séquence ayant 80% ou plus d'homologie avec la séquence représentée par SEQ ID NO : 43.

2. Procédé selon la revendication 1, dans lequel les deux anticorps différents sont une combinaison d'un anticorps ayant des régions CDR identiques aux régions CDR contenues dans une région variable de chaîne lourde représentée dans SEQ ID NO: 38 et des régions CDR contenues dans une région variable de chaîne légère représentée dans SEQ ID NO: 39, et un anticorps ayant des régions CDR identiques aux régions CDR contenues dans une région variable de chaîne lourde représentée dans SEQ ID NO: 40 et des régions CDR contenues dans une région variable de chaîne légère représentée dans SEQ ID NO: 41, ou
une combinaison d'un anticorps ayant des régions CDR identiques aux régions CDR contenues dans une région variable de chaîne lourde représentée dans SEQ ID NO: 44 et des régions CDR contenues dans une région variable de chaîne légère représentée dans SEQ ID NO: 45, et un anticorps ayant des régions CDR identiques aux régions CDR contenues dans une région variable de chaîne lourde représentée dans SEQ ID NO: 42 et des régions CDR contenues dans une région variable de chaîne légère représentée dans SEQ ID NO: 43.

3. Procédé selon la revendication 2, dans lequel les régions CDR sont des régions CDR1, CDR2 et CDR3 basées sur la numérotation Kabat.

4. Procédé selon la revendication 1, dans lequel les deux anticorps différents sont une combinaison d'un anticorps ayant une région variable de chaîne lourde dont les CDR1, CDR2 et CDR3 comprennent les séquences d'acides aminés représentées par SEQ ID NO: 46, 47 et 48, respectivement, et ayant une région variable de chaîne légère dont CDR1, CDR2 et CDR3 comprennent les séquences d'acides aminés représentées par SEQ ID NO: 49, 50 et 51, respectivement, et un anticorps ayant une région variable de chaîne lourde dont CDR1, CDR2 , et CDR3 comprennent les séquences d'acides aminés représentées par SEQ ID NO: 52, 53 et 54, respectivement, et ayant une région variable de chaîne légère dont CDR1, CDR2 et CDR3 comprennent les séquences d'acides aminés représentées par SEQ ID NO: 55, 56 et 57, respectivement, ou
une combinaison d'un anticorps ayant une région variable de chaîne lourde dont CDR1, CDR2 et CDR3 comprennent les séquences d'acides aminés représentées par SEQ ID NO: 58, 59 et 60, respectivement, et ayant une région variable de chaîne légère dont CDR1, CDR2 et CDR3 comprennent les séquences d'acides aminés représentées par SEQ ID NO: 61, 62 et 63, respectivement, et un anticorps ayant une région variable de chaîne lourde dont CDR1, CDR2 et CDR3 comprennent les séquences d'acides aminés représentées par SEQ ID NO: 64, 65 et 66, respectivement, et ayant une région variable de chaîne légère dont CDR1, CDR2 et CDR3 comprennent les séquences d'acides aminés représentées par les SEQ ID NO: 67, 68 et 69, respectivement.

5. Procédé selon la revendication 1, dans lequel les deux anticorps différents sont une combinaison d'un anticorps ayant une région variable de chaîne lourde représentée dans SEQ ID NO: 38 et une région variable de chaîne légère représentée dans SEQ ID NO: 39, et un anticorps ayant une région variable de chaîne lourde représentée dans SEQ ID NO: 40 et une région variable de chaîne légère représentée dans SEQ ID NO: 41, ou
une combinaison d'un anticorps ayant une région variable de chaîne lourde représentée dans SEQ ID NO: 44 et une région variable de chaîne légère représentée dans SEQ ID NO: 45, et un anticorps ayant une région variable de chaîne lourde représentée dans SEQ ID NO: 42 et une région variable de chaîne légère représentée dans SEQ ID NO: 43.

6. Procédé selon l'une quelconque des revendications 1 à 5, dans lequel l'un quelconque des deux anticorps différents est lié à une particule magnétique.

7. Procédé selon l'une quelconque des revendications 1 à 6, dans lequel l'échantillon à tester est un échantillon de tissu, un échantillon de sang total, un échantillon de plasma ou un échantillon de sérum isolé d'un humain.
